# EUROPEAN PATENT APPLICATION

(11) **EP 4 455 121 A1**
(43) Date of publication of application: **30.10.2024**
(21) Application number: 22911392.3
(22) Date of filing: 23.12.2022
(51) Int. Cl.: C07C 69/007, A61K 8/89, A61Q 1/12, A61Q 17/04, C08F 290/14, C08G 63/91, C08G 77/46, C08L 101/16

(54) **REACTIVE GROUP-INCLUDING POLYCAPROLACTONE COMPOUND, NOVEL SILICONE ELASTOMER PARTICLES IN WHICH SAME IS USED, COSMETIC COMPOSITION, AND OTHER APPLICATION**

(30) Priority: 24.12.2021 JP 2021210861
(71) Applicant: Dow Toray Co., Ltd., Tokyo 140-8617 (JP)
(72) Inventor: SUGIURA Tsunehito, Ichihara-shi Chiba 299-0108 (JP); WAKITA Mari, Ichihara-shi Chiba 299-0108 (JP); TANIGUCHI Hiroko, Ichihara-shi Chiba 299-0108 (JP); TAN Liyi, Ichihara-shi Chiba 299-0108 (JP); KANZAKI Yasue, Ichihara-shi Chiba 299-0108 (JP); NAGAYAMA Kei, Ichihara-shi Chiba 299-0108 (JP); SETOGUCHI Hiro, Ichihara-shi Chiba 299-0108 (JP)
(74) Representative: Murgitroyd & Company
(86) International application number: PCT/JP2022/047560
(87) International publication number: WO 2023/120690

(57) **Abstract**

PROBLEM

To provide silicone elastomer particles having a crosslinked structure which is active with respect to biodegradability, and can provide texture and feel during use that is equal to or higher than conventional silicone elastomer particles when blended in a cosmetic composition or the like, a reactive group-containing polycaprolactone compound having a specific structure and useful as a raw material for synthesis reactions, and a method for producing the same.

SOLUTION

A polycaprolactone compound containing a reactive group having two or more modified polycaprolactone structures having the following structural formula (1): {in the formula, n is a number in the range 1 to 5 and Ra is a specific (meth)acryl terminal group or alkenyl terminal group} (specifically, a compound selected from the aforementioned (meth)acryl-modified polycaprolactone compounds and alkenyl-modified polycaprolactone compounds) and their use as raw materials for silicone elastomer particles.

## Description

### TECHNICAL FIELD

The present invention provides a polycaprolactone compound containing reactive groups useful as a raw material for synthesis reactions selected from radical polymerization and hydrosilylation reactions, as well as a production method thereof. Furthermore, the present invention relates to a novel silicone elastomer particle having a crosslinked structure between silicon atoms derived from the reactive group polycaprolactone compound, and capable of imparting excellent texture and feel during use to a cosmetic material. Furthermore, the novel silicone elastomer particles have a crosslinked structure that is active with respect to biodegradability. Therefore, primary particles thereof are expected to have a property of disintegrating with the generation of non-crosslinked siloxane molecules due to a decomposition reaction of microorganisms and the like in the natural world, and thus are expected to behave as biodegradable silicone elastomer particles. Furthermore, the present invention relates to a cosmetic raw material, cosmetic material composition, organic resin additive, and other applications containing the silicone elastomer particles, as well as to a method of manufacturing the silicone elastomer particles.

### BACKGROUND ART

Silicone elastomer particles are cured from an addition reaction-curable or condensation reaction-curable silicone composition, and the particle diameter and oil absorbency thereof vary depending on the manufacturing method, but are widely used as stress-relieving agents or the like for cosmetic raw materials and thermoplastic resins. For example, as silicone particles having superior dispersibility, high lipophilicity, and superior storage stability, the present applicant has proposed silicone particles containing an alkylene group having 4 to 20 carbon atoms, which is obtained by curing a crosslinkable composition for forming silicone particles having a low amount of silicon atom-bonded hydrogen atoms per unit mass and containing an alkenyl group having 4 to 20 carbon atoms, such as a hexenyl group or the like, as described in Patent Document 1.

In addition, the applicant has focused on essential issues in conventional silicone elastomer particles. In other words, conventional silicone elastomer particles are formed through a crosslinking reaction of organopolysiloxane raw materials by hydrosilylation reactions or the like. The crosslinked structure is chemically stable, and even if these silicone elastomer particles were to be released into nature, it is undeniable that they would remain in nature without decomposing, at least for a short period of time, just like so-called microplastics. Therefore, there seems to be a latent desire in the market for silicone elastomer particles that perform well enough to smoothly replace or substitute existing silicone elastomer particles and that are expected to be highly biodegradable, in order to reduce risk to the global environment.

In view of this potential market demand, the present applicants have proposed a silicone elastomer particle having a structure crosslinked by a divalent organic group having a partial structure formed by radical polymerization of vinyl acetate as described in Patent Document 2. The silicone elastomer particles can be expected to have a high degree of biodegradability, have suppressed aggregation properties over time as compared with conventional silicone elastomer particles, and provide a smaller average secondary particle diameter. Therefore, these silicone elastomer particles have excellent dispersibility, and have excellent handling workability as a cosmetic raw material, storage stability, and blending stability in a system.

However, there is still a need for silicone elastomer particles that can impart a feel equal to or better than that of existing silicone elastomer particles and that can be expected to have high biodegradability when used as a cosmetic raw material.

On the other hand, a polycaprolactone compound is synthesized by ring-opening addition polymerization of ε-caprolactone, and is expected to have properties as a biodegradable raw material in addition to being a film-forming material. Patent Document 3 discloses an (AB)n type block copolymer containing a polycaprolactone structure and a polysiloxane structure. However, there is no description or suggestion of silicone elastomer particles having the polycaprolactone structure, and there is no disclosure of a (meth)acrylic-modified polycaprolactone compound having a terminal group expressed by -C(=O)-CH=CH₂ or -C(=O)-CH=CH(CH₃).

On the other hand, Non-patent Documents 1 to 3 disclose reacting a polycaprolactone compound having a polyol terminal structure with butyroyl chloride or the like, but do not disclose a (meth)acryl-modified polycaprolactone compound having a specific structure, which has a plurality of polycaprolactone structures having a relatively low degree of polymerization in each molecule, all of the terminal structures being (meth)acryl terminal groups, and can form silicone elastomer particles by a crosslinking reaction between polysiloxane structures or a radical polymerization reaction with a (meth)acryl group-containing organopolysiloxane.

### RELATED ART DOCUMENTS

### PATENT DOCUMENTS

Patent Document 1: International Patent Publication WO2017/191798
Patent Document 2: International Patent Application PCT/JP 2021/46142
Patent Document 3: Japanese Unexamined Patent Application 2002-146026 A (Patent No. 3512399)

### [NON-PATENT DOCUMENTS]

Non-patent Document 1: Preparation of positively charged polycaprolactone film material and application of biomaterial (IWAMATSU Koji et al., Proceedings of the 2017 Nihon University Science & Engineering Department, pp. 1125 to 1126)
Non-patent Document 2: Temperature-responsive cross-linked poly ((-caprolactone) membrane that functions near body temperature (Koichiro Uto et al., Journal of Controlled Release 110 (2006) 408 to 413).
Non-patent Document 3: A novel degradable polycaprolactone network for tissue (written by HaeYong Kweon et al., Biomaterials 24 (2003) 801 to 808).

### SUMMARY OF THE INVENTION

### PROBLEM TO BE SOLVED BY THE INVENTION

An object of the present invention is to solve the aforementioned problems, and provide silicone elastomer particles having a crosslinked structure which is active with respect to biodegradability, and can provide texture and feel during use that is equal to or higher than conventional silicone elastomer particles when blended in a cosmetic composition or the like, a reactive group-containing polycaprolactone compound having a specific structure and useful as a raw material for synthesis reactions, and a method for producing the same.

Furthermore, an object of the present invention is to provide a cosmetic raw material, an organic resin additive, and other applications with excellent feel during use, and the like by using the silicone elastomer particles. Furthermore, an object of the present invention is to provide a cosmetic material composition containing the silicone elastomer particles, with excellent feel during use, and the like.

Furthermore, an object of the present invention is to provide: silicone elastomer particles that, in addition to performance equal to or better than conventional silicone elastomer particles, can be expected to be biodegradable, thereby reducing potential risks to the global environment, allowing for industrially sustainable and stable use, and making it possible to promote the material as biodegradable and eco-friendly to users and consumers in general who are concerned about global environmental impact. Also to provide a synthesis material, and a use thereof.

### MEANS FOR SOLVING THE PROBLEM

As a result of intensive studies to solve the above problems, the present inventors have found that the problems can be resolved by using a reactive group-containing polycaprolactone compound having 2 or more modified polycaprolactone structures expressed by the following structural formula (1): 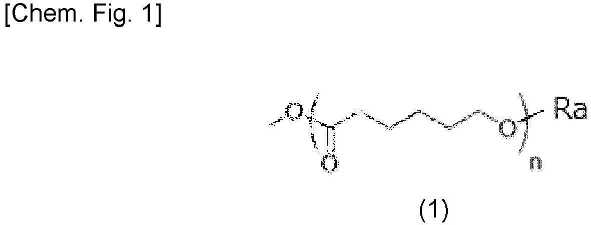 {where n is a number in the range of 1 to 5, Ra is a (meth)acryl terminal group expressed by

-C(=O)-R¹-CR²=CH₂

(R¹ is a carbonyl group or a divalent linking group containing one carbonyl group, and R² is a hydrogen atom or a methyl group) or an alkenyl terminal group.} or by using this as a silicone elastomer particle raw material, and thus the present invention was achieved. Note that the aforementioned reactive group refers to one or more reactive functional group selected from (meth)acryl-modified groups and alkenyl-modified groups having radical polymerizability or hydrosilylation reactivity, or in other words, the reactive group-containing polycaprolactone compound according to the present invention includes both a (meth)acryl-modified polycaprolactone compound and an alkenyl-modified polycaprolactone compound.

Similarly, the present inventors found that the above problems can be solved by silicone elastomer particles having a structure in which at least two silicon atoms in the silicone elastomer particles are crosslinked by one or more reactions selected from radical polymerization reactions and hydrosilylation reactions of the reactive group-containing polycaprolactone compound, more specifically, the radical polymerization reaction of a (meth)acryl-modified polycaprolactone compound and the hydrosilylation reaction of an alkenyl-modified polycaprolactone compound with a silicon-bonded hydrogen atom, as well as by cosmetic raw materials, organic resin additives, cosmetic materials, and organic resins containing the same, thus achieving the present invention.

### EFFECT OF THE INVENTION

When the silicone elastomer particles obtained by using the reactive group-containing polycaprolactone compound according to the present invention as a raw material are blended in a cosmetic material composition or the like, it is possible to achieve a texture and a feel during use which are equal to or higher than those of conventional silicone elastomer particles. Furthermore, the silicone elastomer particles according to the present invention can be used to provide a cosmetic raw material, an organic resin additive, and other applications containing the silicone elastomer particles. Furthermore, a cosmetic material composition containing the silicone elastomer particles according to the present invention can provide a cosmetic material with excellent feel during use, and the like.

Furthermore, the silicone elastomer particles of the present invention have a structure crosslinked by a divalent organic group having a partial structure formed by a radical polymerization or hydrosilylation reaction of the reactive group-containing polycaprolactone compound of the present invention between at least 2 silicon atoms constituting the polyorganosiloxane chain in the silicone elastomer particles, and the divalent organic group having this partial structure is active in a biodegradable reaction, and the crosslinked structure formed between silicon atoms in the silicone elastomer particles is at least partially cleaved in a biodegradable environment, and the primary particles of the silicone elastomer particles are designed to have the property of being disintegrated with the generation of a polyorganosiloxane having a non-crosslinked structure. Therefore, the silicone elastomer particles according to the present invention are expected to be biodegradable, which reduces the risk to the global environment, and appeals to users and consumers in general, who are concerned about global environment impact, as an eco-friendly material that can be used with a considerable sense of ease.

### BRIEF DESCRIPTION OF THE DRAWINGS

FIG. 1 is an image of silicone elastomer particles No. 1 (Example 5) according to the present invention observed with a digital microscope (device name: KEYENCE model number VH-6000).
FIG. 2 is an image of silicone elastomer particles No. 3 (Example 7) according to the present invention observed with a digital microscope (device name: KEYENCE model number VH-6000).
FIG. 3 depicts the results of an enzymatic degradation test of the silicone elastomer particles of Examples 8, 7, and 12 according to the present invention, and the comparative test results of the conventional silicone elastomer product (comparative test).

### PREFERRED MODE FOR CARRYING OUT THE INVENTION

In the present specification, the term "(meth)acryl" refers to "acrylic or methacrylic", and when expressed as "(meth)acryl modified" indicates that the modifying group may be one or both of an acryl modifying group and a methacryl modifying group. Similarly, the term "(meth) acryloxy" refers to "methacryloxy or acryloxy" and "(meth)acryloxy group-containing organic group" indicates either one or both of methacryloxy group-containing organic groups and acryloxy group-containing organic groups.

### [Reactive Group-Containing Polycaprolactone Compound]

The reactive group-containing polycaprolactone compound according to the present invention is one or more compounds selected from (meth)acryl-modified polycaprolactone compounds and alkenyl-modified polycaprolactone compounds, is designed as a reactive raw material for the novel silicone elastomer particles according to the present invention, and is a component that provides a crosslinked structure by a divalent organic group having a specific partial structure between at least two silicon atoms constituting a polyorganosiloxane chain in the silicone elastomer particles by a radical polymerization reaction and a hydrosilylation reaction with silicon-bonded hydrogen atoms.

Specifically, the reactive group-containing polycaprolactone compound of the present invention has two or more modified polycaprolactone structures expressed by the following structural formula (1) in the molecule: 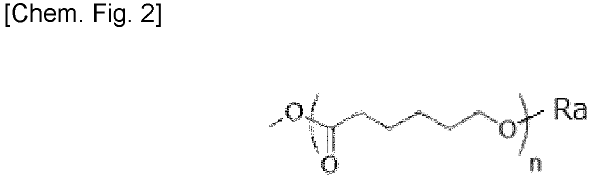
(1)This compound has two or more (meth)acryl-modified groups or alkenyl-modified groups at the terminals, so it is expected that when a crosslinked structure is formed in the silicone elastomer particles by a hydrosilylation reaction or radical polymerization reaction and the resulting silicone elastomer particles are used as a cosmetic raw material, the feel of use and texture will not be impaired, and the crosslinked structure formed between the two silicon atoms will have biodegradability.

Here, n in the formula represents the number of repeating caprolactone units {-C(=O)-C₅H₁₀-O-} in the structure, and is a number in a range of 1 to 5, and may be a number in a range of 1 to 3. The reactive group-containing polycaprolactone compound of the present invention is designed as a crosslinking agent between silicon atoms in the silicone elastomer particles, has a relatively small number of caprolactone units in each structure, and has a relatively small number of repeating units of caprolactone units in the molecule as a whole, and therefore has the advantage of not significantly impairing the texture and feel during use derived from the organopolysiloxane main chain of the silicone elastomer particles.

In the formula, the (meth)acryl terminal group or alkenyl terminal group is expressed by -C(=O)-R¹-CR²=C₂. Here, R¹ is a carbonyl group or a divalent linking group containing one carbonyl group, and is preferably a group selected from carbonyl groups (-C(=O)-), a divalent organic group having 1 to 20 carbon atoms containing a carbonyl group, or a divalent silicon atom-containing group containing a carbonyl group. Furthermore, R² is a hydrogen atom or a methyl group, and provides an acryl-modified group, a methacryl-modified group, or an alkenyl-modified group.

The reactive group-containing polycaprolactone compound of the present invention is a crosslinking agent between at least two silicon atoms, so it must have at least two reactive groups selected from reactive (meth)acryl-modified groups and alkenyl-modified groups in the molecule. Therefore, the (meth) reactive group-containing polycaprolactone compound of the present invention is required to have two or more of the above structures in the molecule, and may have 2 to 4 of the above structures. This is because a polycaprolactone compound having a polyolic (alcoholic) hydroxyl group terminal which is a precursor of the structure is relatively easily available as a reactive production raw material on an industrial scale.

In the reactive group-containing polycaprolactone compound of the present invention, in addition to the fact that the number of repeating caprolactone units in each structure is relatively small, the sum of the numbers of repeating caprolactone units in the molecule is preferably in a range of 2 to 20, and may be in a range of 2.5 to 15, or in a range of 3.0 to 12. If the number of repeating caprolactone units in the reactive group-containing polycaprolactone compound molecule exceeds the upper limit, the properties derived from the polycaprolactone structure are strongly reflected in the obtained silicone elastomer particles, which may adversely affect the texture and feel during use of the cosmetic material or the like.

More specifically, the reactive group-containing polycaprolactone compound of the present invention may be a compound expressed by one or more structural formulas selected from the following structural formulas (1-1) to (1-5). These compounds have two, three, or four modified polycaprolactone structures expressed by the following structural formulas (1).

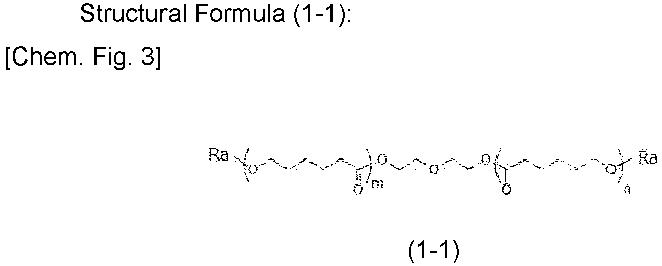

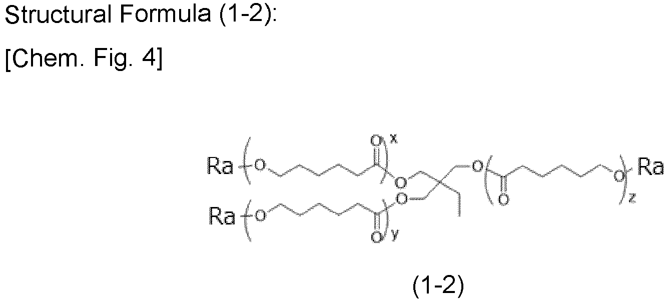

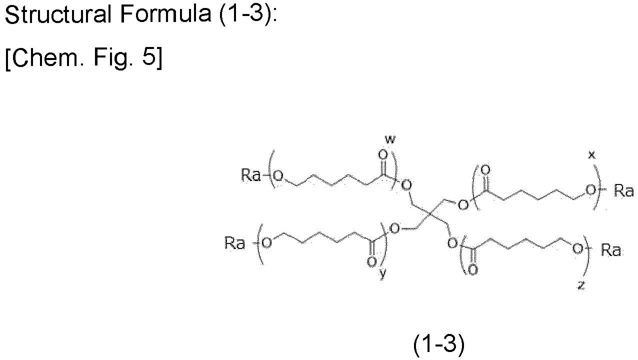

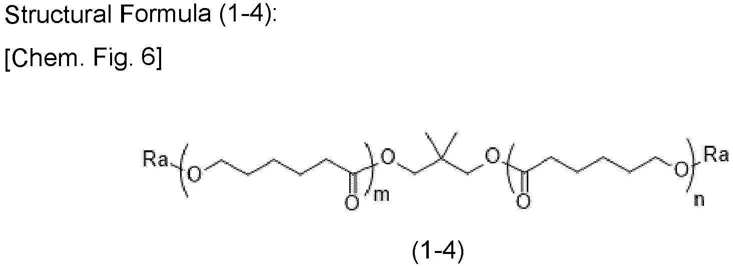

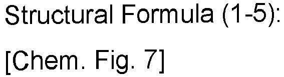

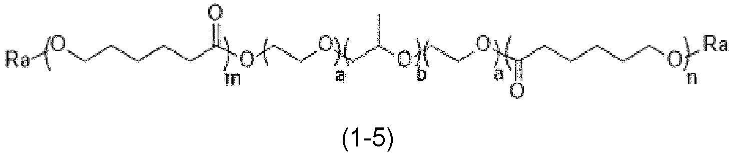

In the formula, Ra is synonymous with the groups described above.
In structural formula (1-1), "n" is a number in a range of 1 to 2, while "m" is a number in a range of 2 to 20, and may be a number in a range of 2 to 10, while m + n is a number in a range of 2 to 20, or m + n is a number in a range of 2.5 to 15, or can be a number in a range of 3 to 10.
In structural formulas (1-2) and (1-3), w, x, y, and z may each independently be a number in a range of 1 to 5, x + y + z may be a number in a range of 3 to 20, and w + x + y + z may be a number in a range of 4 to 20. Furthermore, x + y + z may be a number in a range of 3 to 7, and w + x + y + z may be a number in a range of 4 to 10.
In structural formula (1-4), "n" is a number in a range of 1 to 2, while "m" is a number in a range of 2 to 20, and may be a number in a range of 2 to 10, while m + n is a number in a range of 2 to 20, or m + n is a number in a range of 2.5 to 15, or can be a number in a range of 3 to 10.
In structural formula (1-5), "n" is a number in a range of 1 to 2, while "m" is a number in a range of 2 to 20, and may be a number in a range of 2 to 10, while m + n is a number in a range of 2 to 20, or m + n is a number in a range of 2 to 15, or can be a number in a range of 2 to 10. Furthermore, a + b may be a number in a range of 1 to 500, and a + b may be a number in a range of 2 to 350.

The reactive group-containing polycaprolactone compound, more specifically, a compound selected from a (meth)acryl-modified polycaprolactone compound and an alkenyl-modified polycaprolactone compound can be obtained by reacting a polycaprolactone compound having a polyol terminal structure as a precursor with a (meth)acryloyl chloride compound and an alkenoyl chloride compound in the presence of a basic catalyst, or can be obtained by reacting tetramethyldivinyldisilazane (hereinafter sometimes referred to as "vinylsilazane") with an acidic catalyst.

Specifically, the reactive group-containing polycaprolactone compound of the present invention has the following structural formula (1') in the molecule: 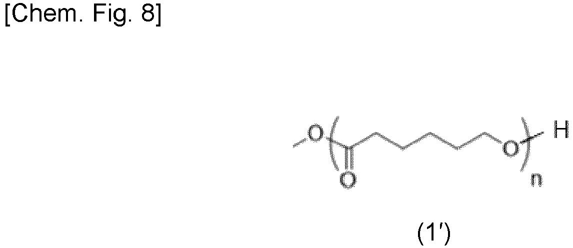 (where n represents a number within a range of 1 to 5) with the compound can be obtained by reacting a (meth)acryloyl chloride compound expressed by

Cl-C(=O)-R¹-CR²=CH₂

(R¹ is a chemical bond between CH and C(=O) or a divalent organic group having 1 to 20 carbon atoms, and R² is a hydrogen atom or a methyl group) with an alkenoyl chloride compound in the presence of a basic catalyst, and further by reacting vinylsilazane with an acid catalyst. The reaction ratio of the polycaprolactone compound, the (meth)acryloyl chloride compound, the alkenoyl chloride compound, and the vinylsilazane is an amount (number of moles) such that the amount of the (meth)acryloyl chloride compound, the alkenoyl chloride compound, and the vinylsilazane is 1 equivalent to a small excess with respect to the amount (number of moles) of the polyol terminal structure (-OH) of the polycaprolactone compound.

The basic catalyst that can be used in the reaction is not particularly limited, and may be an alkali metal salt of an inorganic base such as sodium hydroxide, potassium hydroxide, calcium hydroxide, sodium carbonate, potassium carbonate or sodium hydrogen carbonate; an amine compound such as triethylamine, pyridine or dimethylaminopyridine; or a nitrogen-containing heterocyclic compound. Usable acidic catalysts include, but are not limited to, trifluoromethanesulfonic acid, sulfuric acid, hydrochloric acid and the like.

Examples of organic solvents that can be used in the reaction include ketones such as acetone, methyl ethyl ketone, methyl isobutyl ketone and cyclohexanone; amides such as formamide, acetamide, N-methyl-2-pyrrolidone (NMP), N,N-dimethylformamide, and dimethylacetamide; halogenated hydrocarbons such as methylene chloride, chloroform, 1,2-dichloroethane, chlorobenzene, bromobenzene, dichlorobenzene, benzotrifluoride and hexafluoro-2-propanol; sulfoxides such as dimethyl sulfoxide (DMSO), diethyl sulfoxide and benzylphenyl sulfoxide; ethers such as diethyl ether, diisopropyl ether, dibutyl ether, tetrahydrofuran (THF), dioxane, 1,2-dimethoxyethane and cyclopentylmethyl ether; esters such as ethyl acetate; nitriles such as acetonitrile and benzonitrile; aromatic hydrocarbons such as benzene, toluene and xylene; and mixtures of two or more of these.

In the reaction, one or more polymerization inhibitors may be contained in the system in order to prevent the synthesized reactive group-containing polycaprolactone compound (for example, a (meth)acryl-modified polycaprolactone compound) from further undergoing a radical polymerization reaction. Examples include one or more types selected from hindered phenol-based polymerization inhibitors, hydroquinone-based polymerization inhibitors, and catechol-based polymerization inhibitors.

The reaction conditions should be appropriately selected based on the synthesis amount, the reaction apparatus, and the like, but the (meth)acryloyl chloride compound, the alkenoyl chloride compound, and the vinylsilazane are added dropwise while stirring a mixed solution containing the polycaprolactone compound, the basic catalyst, and the optional polymerization inhibitor under a flow of an inert gas such as nitrogen. Note that after completion of the reaction, it is particularly preferable to mutually separate the intended reactive group-containing polycaprolactone compound and the vinylsilazane, and to distill off the unnecessary organic solvent under reduced pressure for purification.

### [Silicone elastomer particles]

The silicone elastomer particles of the present invention, applications thereof including cosmetic raw materials in particular, a manufacturing method thereof, as well as a cosmetic material composition and an organic resin (including paint and coating agent) containing the same will be described in detail below.

The silicone elastomer particles of the present invention are characterized in that at least two silicon atoms in the silicone elastomer particles have a crosslinked structure due to 1 or more type of reaction selected from the group consisting of
(i) a radical polymerization reaction of the (meth)acryl-modified polycaprolactone compound, which is a reactive group-containing polycaprolactone compound; and
(ii) a hydrosilylation reaction of the aforementioned alkenyl-modified polycaprolactone compound, which is a reactive group-containing polycaprolactone compound, having a silicon-bonded hydrogen atom.

More specifically, the silicone elastomer particles of the present invention are obtained by a crosslinking reaction selected from a radical polymerization reaction and a hydrosilylation reaction, and each of these have the following structural features.

### [Radical polymerization-reactive silicone elastomer particles]

The silicone elastomer particle is obtained by radically polymerizing an organopolysiloxane having three or more silicon-bonded radical reactive functional groups such as a (meth)acryloxy group-containing organic group bonded to a silicon atom in the molecule and a (meth)acryl-modified polycaprolactone compound in the presence of a radical polymerization initiator, and has a crosslinked structure formed between at least two silicon atoms in the silicone elastomer particles by a radical polymerization reaction between the terminal (meth)acryl-modified group of the (meth)acryl-modified polycaprolactone compound and the silicon-bonded radically reactive functional group.

### [Hydrosilylation-reactive silicone elastomer particles]

The silicone elastomer particle is obtained by subjecting an organopolysiloxane (= organohydrogenpolysiloxane) having three or more silicon-bonded hydrogen atoms in the molecule and an alkenyl-modified polycaprolactone compound to a hydrosilylation reaction in the presence of a hydrosilylation reaction catalyst, and at least two silicon-silicon bonds in the silicone elastomer particles have a crosslinked structure formed by a hydrosilylation reaction (addition reaction) between a modified group at the alkenyl end of the alkenyl-modified polycaprolactone compound and a silicon-bonded hydrogen atom.

The silicone elastomer particles of the present invention preferably further contain a polyorganosiloxane structure expressed by:

-(R¹₂SiO)ₙ-

(where R¹ represents an unsubstituted alkyl group having 1 to 20 carbon atoms, an alkyl group having 1 to 20 carbon atoms substituted with a halogen atom, an aryl group having 6 to 22 carbon atoms, or a hydroxyl group, and n is a number ranging from 1 to 1000). This is a linear polysiloxane structure derived from component (A), described later, which provides the silicone elastomer particles with an appropriate degree of hardness and flexibility.

Industrially, R¹ preferably independently represents a methyl group or phenyl group, and n is more preferably a number ranging from 50 to 800, and more preferably 75 to 750.

The silicone elastomer particles according to the present invention are preferably obtained by curing crosslinking reactive silicone emulsified particles by a crosslinking reaction. Particularly preferably, the silicone elastomer particles of the present invention have silicone elastomer particles including at least:
(A) at least one type of reactive organopolysiloxane selected from the following component (a1) and component (a2);
   (a1) an organopolysiloxane having at least three organic groups containing a (meth)acryloxy group of at least one type selected from organic groups containing a methacryloxy group and organic groups containing an acryloxy group in each molecule;
   (a2) an organopolysiloxane having at least three silicon atom-bonded hydrogen atoms per molecule;
(B) a reactive group-containing polycaprolactone compound; and
(C) one or more curing agent selected from radical polymerization initiators and hydrosilylation reaction catalysts; and are obtained by performing in water a crosslinking reaction of crosslinkable silicone emulsified particles obtained by emulsifying in water a crosslinkable silicone composition crosslinkable by one or more reactions selected from radical polymerization reactions and hydrosilylation reactions with a silicon-bonded hydrogen atom.

Furthermore, silicone elastomer particles obtained via such a manufacturing process may further improve the appearance, spreadability, and texture of the cosmetic material, particularly when used as a cosmetic raw material, and particles obtained via this manufacturing method tend to be more suitable for solving the problem of the present invention. Thus, one suitable form for achieving the technical effect of the present invention can be and is suitably defined by the manufacturing process.

The silicone elastomer particles according to the present invention are not particularly limited in terms of the average primary particle diameter thereof, but from the perspective of imparting a smooth texture and feel during use to the cosmetic material, not causing appearance defects and the like, storage stability and blending stability as a cosmetic raw material, and the like, the average particle diameter measured by a laser diffraction scattering method is preferably within a range of 0.5 to 20 µm, more preferably within a range of 0.5 to 15 µm. Note that the particle diameter of the silicone elastomer particles can be controlled according to the crosslinking reactive silicone emulsified particles and crushing/classification process of the resulting silicone elastomer particles.

The shape of the silicone elastomer particles according to the present invention includes, for example, spherical, regular spherical shape, elliptical, and irregular shapes, and in particular, spherical and regular spherical shapes are preferred. Spherical silicone elastomer particles can be easily obtained by the method described later, in which the particles are prepared in the form of an aqueous suspension and dried using a vacuum dryer, hot air circulation oven, or spray dryer.

Furthermore, in the present invention, the crosslinking reactive silicone composition used to form the silicone elastomer particles is preferably within a range of 10 to 80 when cured in a sheet form, as measured by a JIS A hardness tester as defined in JIS K6301. If the JIS-A hardness of the rubber sheet measured by curing the crosslinking reactive silicone composition into a sheet form is within the range above, the resulting silicone elastomer particles will have sufficiently low aggregation and will tend to have exceptional flowability, dispersibility, smoothness, silkiness and a soft texture. Furthermore, by selecting the JIS-A hardness described above, it is possible to design or predict to some extent the feel during use, texture, and handling workability when added to a cosmetic material, and to improve stress relaxation properties when added to an organic resin. Note that when the silicone elastomer particles according to the present invention are used as a cosmetic raw material or stress relief agent or the like for organic resins, silicone elastomer particles having a JIS-A hardness in a range of 30 to 80, and particularly 50 to 80, described above are particularly preferably used.

Optionally, the silicone elastomer particles of the present invention may further contain a structure in which some or all of the surface thereof is covered with one or more types selected from organopolysiloxane resins, silica, and other silicone elastomer particles. Such coating may be expected to further reduce aggregation, control oil absorbency, improve texture, and the like.

Optionally, the silicone elastomer particles of the present invention may be mesoporous structures with micropores.

Optionally, the silicone elastomer particles of the present invention may contain an oil agent that is liquid at 40°C. The oil agent can be easily included in the silicone elastomer particles by emulsifying them together in the crosslinking reactive silicone composition described later, and the inclusion of the oil agent may be expected to further reduce aggregation, control oil absorbency, improve texture, and the like.

Optionally, the silicone elastomer particles of the present invention may further have a structure in which at least two silicon atoms configuring the particles are crosslinked by a silalkylene group having 2 to 20 carbon atoms formed by a hydrosilylation reaction involving an alkenyl group having 2 to 20 carbon atoms. These structures can be easily achieved by using an alkenyl group-containing organopolysiloxane, organohydrogen polysiloxane, and hydrosilylation reactive catalyst in combination. Furthermore, for example, by using an alkenyl group having 4 or more carbon atoms, such as a hexenyl group or the like, further reduction of aggregation, control of oil absorbency, and improvement of texture may be expected due to the combination of the silalkylene structure. However, if biodegradability is a primary objective, the silicone elastomer particles of the present invention are preferably substantially free of structures containing a silalkylene group.

### [Crosslinking reactive silicone composition for Use in Formation of Silicone Elastomer Particles]

The silicone elastomer particles of the present invention can be obtained by crosslinking (curing) a crosslinking reactive silicone composition containing the following components by at least one type of reaction selected from a radical polymerization reaction and a hydrosilylation reaction to the silicon atom bonded hydrogen atom.
(A) at least one type of reactive organopolysiloxane selected from the following component (a1) and component (a2);
   (a1) an organopolysiloxane having at least three organic groups containing a (meth)acryloxy group of at least one type selected from organic groups containing a methacryloxy group and organic groups containing an acryloxy group in each molecule;
   (a2) an organopolysiloxane having at least three silicon atom-bonded hydrogen atoms per molecule;
(B) the reactive group-containing polycaprolactone compound
(C) one or more curing agent selected from radical polymerization initiators and hydrosilylation reaction catalysts;

Note that the crosslinking (curing) reaction is one or more type of reaction selected from radical polymerization reactions and a hydrosilylation reactions, and these reactions may be allowed to proceed simultaneously, but from the viewpoint of reaction control, selecting either one to form the silicone elastomer particles is preferable. In other words, the composition may be a composition containing the following two reaction models and components.

### [Composition for forming radical polymerization-reactive silicone elastomer particles]

The crosslinkable silicone composition contains:
(a1) an organopolysiloxane having at least three organic groups containing at least one type of (meth)acryloxy group selected from organic groups containing a methacryloxy group and organic groups containing an acryloxy group in each molecule;
(B1) the (meth)acryl-modified polycaprolactone compound which is a reactive group-containing a polycaprolactone compound; and
(c1) a photoradical polymerization initiator.

### [Composition for forming hydrosilylation-reactive silicone elastomer particles]

(a2) an organopolysiloxane having at least three silicon atom-bonded hydrogen atoms per molecule;
(B2) the alkenyl-modified polycaprolactone compound which is a reactive group-containing polycaprolactone compound; and
(c2) a hydrosilylation reaction catalyst

Component (a1) is an organopolysiloxane having at least three organic groups containing a (meth)acryloxy group in a molecule, the structure of which is not particularly limited and may be one or more type of structure selected from linear, cyclic, reticulate, or partially branched linear structures. However, a linear organopolysiloxane is particularly preferred. The viscosity of component (a) is preferably a viscosity that allows the crosslinkable composition above to be dispersed in water. Specifically, it is preferably in a range of 20 to 100,000 mPa·s at 25°C, and particularly preferably in a range of 20 to 10,000 mPa s.

From the perspective of texture, dispersibility and handling workability of the silicone elastomer particles, component (a1) is preferably a linear organopolysiloxane in which the amount of the dimethylsiloxane units expressed by the formula: (CH₃)₂SiO is 90 mol% or more of all siloxane units other than the siloxane units at molecular terminals. Similarly, from the perspective of improving the oil absorbency of the resulting silicone elastomer particles and the like, a cyclic or chain organopolysiloxane with a low degree of polymerization (degree of polymerization of 3 to 20) may be removed from component (a1) beforehand by stripping or the like.

Furthermore, when the component (a1) is a linear organopolysiloxane, the silicone elastomer particles according to the present invention, when placed in a biodegradable environment, have an advantage that when the silicone elastomer particles are broken down by cleavage of the crosslinked structure, they are more likely to break down into non-crosslinkable, linear organopolysiloxanes, thus reducing the environmental impact and environmental risk.

Component (a1) must have at least three or more organic groups containing a (meth)acryloxy group on average in a molecule to form a crosslinked structure in a radical reaction with component (B1). If only two or fewer organic groups containing a (meth)acryloxy group are present in a molecule on average, a sufficient crosslinked structure may not be formed, and practical silicone elastomer particles may not be obtained.

More specifically, the organic group containing a (meth)acryloxy group is a (meth)acryloxy group bonded to a silicon atom via a divalent organic group, and one or more functional group expressed by the following is exemplified.

-R²-O-C(=O)-C(R³)=CH₂

{where R² represents an alkylene group having 1 to 20 carbon atoms or a divalent linking group expressed by

(CH₂)ₚ-Si(CH₃)₂-O-Si(CH₃)₂-(CH₂)_{q}

(p and q in the formula are each a number ranging from 1 to 20), and
R³ represents a hydrogen atom or a methyl group.}

The alkylene group, which is R² in the formula, may industrially be an alkylene group having 2 to 10 carbon atoms, and examples include propylene groups, butylene groups, hexylene groups, and the like. Furthermore, a divalent linking group expressed by (CH₂)ₚ-Si(CH₃)₂-O-Si(CH₃)₂-(CH₂)_{q} is a divalent linking group having a siloxane converter structure, and industrial examples include linking groups where p and q are each independently a number from 3 to 6.

Suitably, component (a1) is preferably a linear organopolysiloxane expressed by the following structural formula.

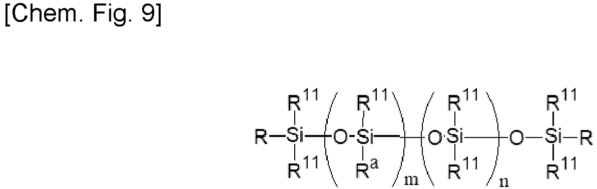

In Formula (1), each R¹¹ is independently an unsubstituted or halogen atom-substituted alkyl group (for example, a methyl group, or the like) having 1 to 20 carbon atoms, an aryl group (for example, a phenyl group, or the like) having 6 to 22 carbon atoms, or a hydroxyl group, and a methyl group or a phenyl group is preferable from an industrial point of view. R^{a} represents an organic group containing a (meth)acryloxy group, and is particularly preferably a (meth)acryloxy group bonded to a silicon atom by the aforementioned alkylene group or divalent linking group with a siloxane converter structure. R independently is a group represented by R¹¹ or R^{a}. m is a number of 1 or more, and n is a number of 1 or more. However, component (a) contains at least three organic groups containing a (meth)acryloxy group represented by R^{a} in a molecule, and therefore, when m = 1, R must both be R^{a}. In other words, the linear organopolysiloxane expressed by the structural formula above has a organic group containing a (meth)acryloxy group represented by R^{a} at one end site and a side chain site, a side chain site only, or both end sites and a side chain site of a siloxane molecule thereof, and preferably contain at least three organic groups containing a (meth)acryloxy group in a molecule.

m + n is the degree of siloxane polymerization of linear organopolysiloxane molecules excluding end siloxane structures. From the perspective of handling workability as a raw material and the ability to break down into fine linear siloxane molecules during emulsification biodegradation, m + n is preferably within a range of 10 to 800, more preferably 20 to 600, and particularly preferably 30 to 500. Furthermore, the viscosity of component (a) is particularly preferably a number between 20 and 10,000 mPa·s at 25°C.

Component (a2) is an organopolysiloxane component that is crosslinked by component (B2) by a hydrosilation reaction, is characterized by having at least three silicon-bonded hydrogen atoms in each molecule, and the bonding positions of the hydrogen atoms in the molecules are not particularly limited.

Examples of an organic group other than a hydrogen atom that is bonded to a silicon atom contained by component (a2), include an alkyl group such as a methyl group, an ethyl group, a propyl group, a butyl group, or an octyl group, and a methyl group is preferable. Examples of the molecular structure of the organohydrogen polysiloxane of component (a2), include straight-chain, branched-chain, and branched-cyclic, or a combination of one or more thereof. The number of silicon-bonded hydrogen atoms in a molecule is the average value of all molecules.

In particular, when the component (a2) is a linear organopolysiloxane (organohydrogenpolysiloxane), the silicone elastomer particles according to the present invention, when placed in a biodegradable environment, have an advantage that when the silicone elastomer particles are broken down by cleavage of the crosslinked structure, they are more likely to break down into non-crosslinkable, linear organopolysiloxanes, thus reducing the environmental impact and environmental risk.

The viscosity of component (a2) at 25 °C is 1 to 1,000 mPa-s, preferably 5 to 500 mPa-s. This is because if the viscosity of component (b) at 25 °C is less than 1 mPa-s, component (a2) is easily volatilized from the crosslinkable composition containing it, and if the viscosity exceeds 1,000 mPa-s, the curing time of the crosslinkable composition containing such component (a2) may become longer or may cause curing failure. Such component (a2) is not particularly limited, and examples include, dimethylsiloxane-methylhydrogensiloxane copolymer capped at both molecular terminals with trimethylsiloxy groups, dimethylsiloxane-methylhydrogensiloxane copolymer capped at both molecular terminals with dimethylhydrogensiloxy groups, dimethylpolysiloxane capped at both molecular terminals with dimethylhydrogensiloxy groups, methylhydrogenpolysiloxane capped at both molecular terminals with trimethylsiloxy groups, cyclic methylhydrogenpolysiloxane, and cyclic methylhydrogensiloxane-dimethylsiloxane copolymer.

Here, the value of H/Alk, which is the molar ratio (= reaction ratio in the hydrosilylation reaction) of the carbon-carbon double bond (Alk) in the alkenyl-modified polycaprolactone compound that is the component (B2) to the silicon atom-bonded hydrogen atom amount (H) in component (a2) is preferably in a range of 0.7 to 1.2. The lower limit of said H/Alk is preferably 0.80 or more, 0.85 or more, 0.90 or more, 0.95 or more, and the upper limit is 1.15 or less, and preferably, 1.10 or less, or 1.05 or less. If the upper limit of H/Alk exceeds the aforementioned value, unreacted silicon-bonded hydrogen atoms tend to remain after the reaction; conversely, if the upper limit of H/Alk is less than the aforementioned value, unreacted component (B) and alkenyl modified terminal groups thereof tend to remain after the reaction. Since these are curing reactive groups, if a large amount of them remain in the particles, they may cause crosslinking reactions between particles over time, resulting in aggregation and poor dispersion of the resulting oil-containing silicone elastomer particles, and if reactive hydrogen atoms remain, they may cause the generation of flammable hydrogen gas over time. Particularly suitably, when the value of H/Alk is in the range of 0.9 to 1.1, particularly around 1.0, the curing reactive groups are completely consumed and the crosslinking reaction is terminated, and the aggregation over time between particles can be effectively suppressed.

Component (B) is a reactive group-containing polycaprolactone compound (specifically, a compound selected from the aforementioned (meth)acryl-modified polycaprolactone compounds and alkenyl-modified polycaprolactone compounds), and provides a crosslinking agent for component (A) which gives the silicone elastomer particles their characteristic crosslinked structure, and a radical polymerizable monomer component that itself forms a polymer or copolymer structure through radical polymerization. The crosslinked portions, whether of the hydrosilylation reaction type or the radical polymerization type, do not significantly impair the feel during use and texture of the resulting silicone elastomer particles, and are active in biodegradable reactions, and in a biodegradable environment, the crosslinked structures formed between the silicon atoms in the silicone elastomer particles are at least partially cleaved, and the primary silicone elastomer particles have the property of disintegrating with the generation of non-crosslinked polyorganosiloxane structures. Herein, if component (A) is a linear polyorganosiloxane as described above, the biodegradable reaction facilitates the breakdown of the silicone elastomer particles into linear polyorganosiloxane molecules, which are broken down into fine liquid components, not solid powders with minute particle diameter sizes, as in macroplastics. Therefore, it is expected to have little impact or burden on the global environment, as it is unlikely to cause problems of bioaccumulation through the food chain or accumulation/deposition in the environment.

The amount of component (B) used must be selected as follows in accordance with the type of component (A) and the crosslinking reaction for obtaining the silicone elastomer particles of the present invention.

When silicone elastomer particles are formed by the radical polymerization reaction of the aforementioned component (a1) and component (B1), the mass ratio of the amount of component (B1) to the amount of the (meth)acryloxy group-containing organic group in component (a1) is in a range of 0.5 to 30, preferably a range of 1 to 20, preferably 3 to 15, and particularly preferably 5 to 10. If the amount of component (B) used is within the range described above, a crosslinked structure derived from (meth)acryl-modified polycaprolactone compound with an appropriate average length can be obtained between the polyorganosiloxane structures, so that the particles can achieve a smooth surface state with moderate hardness and low tack, and the feel during use and texture can be improved. On the other hand, if the amount of component (B) used is less than the lower limit above, crosslinking may be insufficient. If the amount of component (B) used exceeds the upper limit above, emulsion breakdown and the like are likely to occur during a curing reaction, and thus silicone elastomer particles may not be obtained.

When the silicone elastomer particles are formed by the hydrosilation reaction between components (a2) and (B2), the amount of component (B2) is preferably such that the molar ratio of the carbon-carbon double bonds (Alk) contained in the alkenyl-modified terminal groups to the silicon-bonded hydrogen content (H) of component (a2), or in other words, H/Alk, is within the above range.

Component (C) is a curing agent and is selected from (c1) a radical polymerization initiator and (c2) a hydrosilylation reaction catalyst, depending on the selection of component (A) and the reaction system.

Component (c1) is a radical initiator and is a component for promoting a radical polymerization reaction or radical copolymerization reaction of components (a1) and (B1) described above. A conventionally known compound commonly used in radical polymerization methods is used as the radical initiator, and specific examples include 2,2'-azobis(isobutyronitrile), 2,2'-azobis(2-methylbutyronitrile), 2,2'-azobis(2,4-dimethylvaleronitrile), and other azo-based compounds; benzoyl peroxide, lauroyl peroxide, tert-butylperoxy benzoate, tert-butylperoxy-2-ethylhexanoate, tert-hexylperoxy-2-ethylhexanoate, and other organic peroxides; and potassium persulfate, sodium persulfate, ammonium persulfate, and other persulfates. One type of this radical initiator may be used alone, or two or more types may be mixed and used together.

The amount of component (c1), which is a radical initiator, used is preferably within a range of 0.1 to 5 parts by mass relative to 100 parts by mass of the total of components (a1) and (B1) above. In particular, when component (c1) is a water-soluble persulfate such as potassium persulfate or the like, component (c1) has an advantage of being extremely easy to add and react when crosslink reacting in water crosslinking reactive silicone emulsified particles, which are obtained by emulsifying a crosslinking reactive silicone composition in water via a radical polymerization reaction. Furthermore, upon completion of the radical polymerization reaction, it is particularly desirable to add aminomethylpropanediol or the like in a range of 0.1 to 5 parts by mass, for the purpose of stopping the reaction and neutralizing the solution by pH adjustment.

The timing of adding component (c1) to the crosslinkable composition can be selected according to the method of forming the silicone elastomer particles, and may be in the form of adding to the composition in advance, or in the form of supplying component (a1) or component (B) from a different spray line, then adding component (c) to one of the components, and then mixing during spraying. The silicone elastomer particles in the present invention are preferably formed via an aqueous suspension, which is formed via emulsification into water, and component (c1) may be added in advance to the crosslinking reactive silicone composition, or an emulsion containing component (c1) may be added separately to the water.

A chain transfer agent can be optionally added during the polymerization reaction of the crosslinking reactive silicone composition above. Specific examples of the chain transfer agent include mercapto compounds such as 2-mercaptoethanol, butyl mercaptan, n-dodecyl mercaptan, 3-mercaptopropyl trimethoxysilane, polydimethylsiloxanes having a mercaptopropyl group, and the like; and halides such as methylene chloride, chloroform, carbon tetrachloride, butyl bromide, 3-chloropropyl trimethoxysilane, and the like.

Optionally, in the polymerization reaction of the crosslinkable silicone composition, a modified polycaprolactone compound having a (meth)acrylic terminal modifying group and an alkenyl terminal modifying group at one terminal may be used in combination in order to adjust the hardness of the particles. Examples of such a (meth)acryl one terminal modification include Placcel FM1 manufactured by Daicel Corporation.

Component (c2) is a hydrosilylation reaction catalyst, which promotes the addition reaction (hydrosilylation reaction) between the carbon-carbon double bond in the (meth)acryl terminal group present in the above crosslinkable composition and a silicon atom-bonded hydrogen atom. Examples of preferred hydrosilylation reaction catalysts are hydrosilylation reaction catalysts containing platinum metal, such as platinic acid chloride, alcohol-modified platinic acid chloride, olefin complexes of platinic acid chloride, complexes of platinic acid chloride with ketones, complexes of platinic acid chloride with vinyl siloxane, platinum tetrachloride, platinum fine powder, platinum supported on an alumina or silica carrier, platinum black, olefin complexes of platinum, alkenylsiloxane complexes of platinum, carbonyl complexes of platinum, and thermoplastic organic resin powders such as methyl methacrylate resin, polycarbonate resin, polystyrene resin, and silicone resin containing these platinum-based catalysts. In particular, platinum alkenylsiloxane complexes such as a complex of platinum chloride with divinyltetramethyldisiloxane, a complex of platinum chloride with tetramethyltetravinylcyclotetrasiloxane, a platinum divinyltetramethyldisiloxane complex, and a platinum tetramethyltetravinylcyclotetrasiloxane complex can be preferably used. Note, as the catalyst for promoting the hydrosilylation reaction, a non-platinum based metal catalyst such as iron, ruthenium, iron/cobalt, or the like may be used.

The amount of component (c) added to the crosslinkable composition should be a catalytic quantity, and usually, an amount in which the amount of the platinum-based metal contained by component (c) is in the range of 1 to 1,000 ppm relative to the total mass of the crosslinkable composition described above is preferred, and an amount in the range of 5 to 500 ppm is even more preferred. The amount of platinum metal in the silicone elastomer particles may be reduced by the method proposed by the present inventors in Japanese Unexamined Patent Application 2014-122316.

The timing of adding component (c2) to the crosslinkable composition can be selected according to the method of forming the silicone elastomer particles, and may be in the form of adding to the composition in advance, or in the form of supplying component (a2) or component (B) from a different spray line, then adding component (c2) to one of the components, and then mixing during spraying. The oil-containing silicone elastomer particles of the present invention are preferably formed via an aqueous suspension, which is formed via emulsification into water, and component (c2) may be added in advance to the crosslinking reactive silicone composition, or an emulsion containing component (c2) may be added separately to the water.

The crosslinkable silicone composition described above may include a curing retarder represented by a hydrosilylation reaction inhibitor. Examples of such curing retarders are acetylene compounds, enyne compounds, organic nitrogen compounds, organic phosphorus compounds, and oxime compounds. Specific compounds include 2-methyl-3-butyn-2-ol, 3,5-dimethyl-1-hexin-3-ol, 3-methyl-1-pentin-3-ol, 2-phenyl-3-butyn-2-ol, and 1-ethynyl-1-cyclohexanol (ETCH) and other alkyne alcohols; 3-methyl-3-trimethylsiloxy-1-butyn, 3-methyl-3-trimethylsiloxy-1-butyn, 3-methyl-3-trimethylsiloxy-1-pentyn, 3,5-dimethyl-3-trimethylsiloxy-1-hexine, 3-methyl-3-penten-1-yne, and 3,5-dimethyl-3-hexene-1-yne enyne compounds; 1-ethynyl-1-trimethylsiloxycyclohexane, bis(2,2-dimethyl-3-butynoxy)dimethylsilane, methyl(tris(1,1-dimethyl-2-propynyloxy))silane, 1,3,5,7-tetramethyl-1,3,5,7-tetravinylcyclotetrasiloxane, and alkenylsiloxane such as 1,3,5,7-tetramethyl-1,3,5,7-tetrahexenylcyclotetrasiloxane and the like. The amount added is within the range of 0.001 to 5 mass parts per 100 mass parts of component (a), but can be designed as appropriate depending on the type of curing retarder used, the characteristics of the hydrosilylation reaction catalyst used, and the amount used.

The crosslinking reactive silicone composition above may contain one or more polymerization inhibitors from the perspective of preventing unintended side reactions and the like. For example, one or more types selected from hindered phenol-based polymerization inhibitors, hydroquinone-based polymerization inhibitors, and catechol-based polymerization inhibitors may be included. The amount used can be selected as appropriate, but the total concentration of the polymerization inhibitor is preferably 50 ppm by mass or less, and more preferably 30 ppm by mass or less, with respect to the sum of components (A) to (C) above.

The crosslinking reactive silicone composition may include a component other than the components above to the extent that the technical effects of the present invention are not impaired. For example, the composition may include: n-hexane, cyclohexane, n-heptane, or other aliphatic hydrocarbons; toluene, xylene, mesitylene, or other aromatic hydrocarbons; tetrahydrofuran, dipropyl ether, or other ethers; an organic solvent such as acetone, methyl ethyl ketone, methyl isobutyl ketone, other ketones, or the like; a phenolic antioxidant, quinone antioxidant, amine antioxidant, phosphorus antioxidant, phosphite antioxidant, sulfuris antioxidant, thioether antioxidant, or other antioxidant; a triazole light stabilizer, benzophenone light stabilizer, or other light stabilizer; a phosphate ester flame retardant, halogen flame retardant, phosphorus flame retardant, antimony flame retardant, or other flame retardant; one or more antistatic agents including cationic surfactants, anionic surfactants, non-ionic surfactants, and the like; a dye; a pigment; or the like.

The silicone elastomer particles of the present invention may optionally further have: (A) a structure in which part or all of a surface thereof is coated by one or more substance selected from organopolysiloxane resins, silica, and other silicone elastomer particles; (B) a mesoporous structure; (C) a structure containing an oil agent that is liquid at 40°C; and (D) a structure crosslinked by a silalkylene group having 2 to 20 carbon atoms, and a combination of arbitrary components providing these structures may be used. In consideration of biodegradability, a biodegradable oil such as olive oil or the like may be added.

### [Hardness of silicone elastomer]

Although the hardness of the silicone elastomer particles cannot be measured directly, the hardness can be measured indirectly by curing the crosslinkable silicone composition used to form the silicone elastomer particles serving as a raw material thereof. Specifically, the crosslinking reactive silicone composition is cured in a sheet form without emulsifying in water, and the hardness of the silicone elastomer sheet can be measured by a JIS A hardness tester as defined in JIS K6301. The hardness of the silicone elastomer according to the present invention varies depending on the type of the crosslinkable silicone composition, the amount of components (a)/(b) used, and the crosslink density, but is preferably within a range of 10 to 80. In addition, the preferred hardness is as described above.

### [Formation of Silicone Elastomer Particles and Manufacturing Method Thereof]

The method of manufacturing the silicone elastomer particles of the present invention includes a method including a step of curing crosslinking reactive silicone emulsified particles, which are obtained by emulsifying in water with the crosslinking silicone composition used to form the silicone elastomer particles above, in the presence of (C) a curing agent to obtain spherical silicone elastomer particles.

More specifically, the silicone elastomer particles according to the present invention can and preferably are prepared using a manufacturing method including the following steps (I) and (II).
Step (I): a step of forming crosslink reactive silicone emulsified particles by emulsifying in water:
   (A) at least one type of reactive organopolysiloxane selected from the aforementioned component (a1) and component; (a2);
   (B) reactive group-containing polycaprolactone compound; and
   (C) one or more curing agent selected from radical polymerization initiators and hydrosilylation reaction catalysts;
   and forming crosslinkable silicone emulsified particles; and
Step (II):
   a step of curing the crosslinking reactive silicone emulsified particle obtained in step (I) in the presence of (C) a curing agent to obtain silicone elastomer particles.

The crosslinkable silicone composition used to form the silicone elastomer particles can be uniformly mixed using a mixer or other mechanical force. Furthermore, in consideration of biodegradability, dilution with a biodegradable oil such as olive oil is possible.

In this method, silicone elastomer particles can be obtained by emulsifying and curing the aforementioned crosslinkable silicone composition in a surfactant aqueous solution. In addition, the particle diameter can be easily adjusted by adjusting the emulsion particle diameter. Examples of these surfactants include nonionic, anionic, cationic, betaine types, as well as water soluble polymers such as polyvinylalcohols and the like. The particle diameter of the resulting silicone elastomer particles varies depending on the type and content of the surfactant. In order to prepare silicone elastomer particles having a small particle diameter, the amount of the surfactant added is preferably within a range of 0.5 to 50 parts by mass with respect to 100 parts by mass of the crosslinkable silicone composition.

An emulsifier is preferably used to uniformly disperse the crosslinking silicone composition in water in the form of crosslinking reactive silicone emulsified particles. Examples of the emulsifying machine include a homomixer, a paddle mixer, a Henschel mixer, a homo-disper, a colloid mill, a propeller agitator, a homogenizer, an in-line continuous emulsifier, an ultrasonic emulsifier, and a vacuum kneading machine.

The aqueous dispersion of the crosslinking reactive silicone emulsified particles prepared by the method above can then be heated or left at room temperature to cure the crosslinking reactive silicone emulsified particles in the aqueous dispersion to prepare an aqueous dispersion of silicone elastomer particles. When such an aqueous dispersion is heated, the heating temperature is preferably 100°C or lower from the perspective of hydrosilylation reactivity or radical polymerization reactivity, and is particularly preferably 10 to 95°C. Furthermore, examples of a method of heating the aqueous dispersion containing the crosslinking reactive silicone emulsified particles include a method of directly heating the water-based dispersion, and a method of adding the aqueous dispersion to hot water. The crosslinking reaction causes the liquid crosslinking reactive silicone emulsified particles to cure in water, forming a aqueous dispersion of silicone elastomer particles.

The resulting silicone elastomer particles of the present invention can be used as-is as an aqueous dispersion (aqueous suspension). In particular, the aqueous suspension form may be and is preferably used in cosmetic raw materials and the like. When added to a cosmetic material that uses an aqueous solution as a dispersion medium (such as hair cosmetic materials and the like), the silicone elastomer particles may be easily and uniformly dispersed to achieve a desired performance and feel during use by being added as an aqueous dispersion containing the silicone elastomer particles of the present invention.

Suitably, the silicone elastomer particles according to the present invention can be isolated by removing water from the aqueous dispersion of silicone elastomer particles. The method of removing water from the aqueous dispersion includes, for example, drying using a vacuum dryer, a hot air circulation oven, or a spray dryer. Note that the heating and drying temperature of the spray dryer must be set appropriately based on the heat resistance, crosslinking temperature, and the like of the silicone elastomer particles. Note that in order to prevent secondary aggregation of the resulting microparticles, the temperature of the silicone elastomer particles is preferably controlled to be equal to or lower than the glass transition temperature thereof. The silicone elastomer particles thus obtained can be recovered by a cyclone, a bag filter, or the like. Note that as a pretreatment for this operation, the dispersion may be concentrated by a method of heating and dehydration, filtration separation, centrifugation, decantation, or the like, and if necessary, the dispersion may be washed with water.

The silicone elastomer particles of the present invention may be surface treated, if necessary, to further improve the aggregation suppression effect of the silicone elastomer particles of the present invention. Furthermore, surface treatment with another known hydrophilic or hydrophobic treatment agent or the like may be applied. Optionally, the resulting silicone elastomer particles may be further coated with silica or other inorganic microparticles, silicone resin, or the like, in whole or in part on the surface thereof, as described above. Furthermore, the resulting silicone elastomer particles may be crushed or pulverized by a mechanical force if necessary, or classified using a known technique.

### [Cosmetic Raw Material and Cosmetic Material Composition]

The silicone elastomer particles of the present invention are useful as cosmetic raw materials, and when blended in a cosmetic composition or the like, the particles are flexible and improve the feel and texture of the cosmetic materials, and the like, and provide outstanding handling workability, storage stability, and blending stability in systems as a cosmetic raw material.

In particular, the silicone elastomer particles of the present invention are superior to known silicone particles in terms of feel during use and texture, have a higher degree of freedom in formulation design, do not absorb oily ingredients over time when formulated in cosmetic materials, do not cause thickening or changes in texture, and when applied to the skin or hair, do not make the cosmetic material feel greasy or sticky. In addition, when the silicone elastomer particles of the present invention are used in combination with a UV protection component, the UV protection effect of the cosmetic material may be improved without impairing the texture and feel during use of the cosmetic material as compared with other powders or existing silicone elastomer particles.

Furthermore, the silicone elastomer particles of the present invention are active with respect to biodegradable reactions while providing performance that is equal or superior to conventionally known silicone elastomer particles. Moreover, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure. Therefore, the silicone elastomer particles are a low risk and low impact material for the global environment. Furthermore, use is possible as a replacement to conventionally known silicone elastomer particles, making it extremely versatile.

The cosmetic material composition containing the silicone elastomer particles of the present invention are not particularly limited in type, and examples include products such as soaps, body shampoos, facial cleansing creams, and other cleaning cosmetic materials; toners, creams/milky lotions, packs, and other base cosmetic products; powders, foundation, and other base makeup cosmetic materials; lipstick, cheek rouge, eye shadow, eyeliners, mascara, and other facial cosmetic materials; nail polish and other makeup cosmetic materials; shampoos, hair rinses, hairdressing material, hair growth promoters, hair dye, and other hair cosmetic materials; perfume and eau de cologne, and other aromatic cosmetic materials; toothpastes; bath agents; and hair removal agents, shaving lotions, antiperspirants/deodorants, sunscreen agents, and other special cosmetic materials. Examples of the dosage forms of these cosmetic material compositions include aqueous liquid, oil-based liquid, emulsion, cream, foam, semi-solid, solid, and powder. These cosmetic material compositions can also be used by spraying.

In these cosmetic material compositions, the amount of the silicone elastomer particles described above is preferably within a range of 0.5 to 99.0 mass% in the cosmetic material composition, and particularly preferably within a range of 1.0 to 95 mass%. This is because if the amount of the silicone elastomer particles described above exceeds the upper limit of the range above, the effect as a cosmetic material is lost, and if the amount is below the lower limit of the range above, feel during use and the like of the cosmetic material composition and the like is less likely to be improved.

With respect to cosmetic material compositions (in particular, each formulation example) containing silicone particles (silicone rubber powder and the like) or silicone composite particles, which have been proposed in (Japanese Unexamined Patent Application H07-316014), (International Patent Publication WO2017/191798), and (Japanese Unexamined Patent Application H02-243612) as well as Japanese Unexamined Patent Application 2011-105663, Japanese Unexamined Patent Application 2011-168634, Japanese Unexamined Patent Application 2011-102354, and Japanese Unexamined Patent Application 2014-122316 described above, the silicone elastomer particles of the present invention can be used in place of a part or all of these silicone-based particles, and there are cases where the feel during use as well as the production efficiency of the cosmetic material compositions proposed in these Patent Documents can be further improved. Note that it goes without saying that examples of cosmetic material compositions containing silicone particles (silicone rubber powder or the like) or silicone composite particles that can be blended with the silicone elastomer particles of the present invention are not limited to the above, and formulations may be designed in which some or all of silicone particle components in commercially available cosmetic materials are replaced by the silicone elastomer particles of the present invention, using a technique common to a person of ordinary skill in the art.

Furthermore, the silicone elastomer particles of the present invention can be applied to replace some or all of these silicone-based particles with respect to the applications and formulations of cosmetic material compositions disclosed in the aforementioned Patent Documents and the like, and the use therein is encompassed within the scope of the invention of the present application. As an example, the silicone elastomer particles of the present invention may be and are preferably used in combinations of arbitrary components, such as cosmetic product media (aqueous media or oil-based media), oil-based media (including oil agents and volatile oil agents), water, colorants, pigments, ultraviolet light blocking components, alcohols, water-soluble polymers, film-forming agents, oil agents, oil-soluble gelling agents, organically modified clay minerals, surfactants, resins, salts, moisturizers, preservatives, antimicrobial agents, antioxidants, pH adjusters, chelating agents, refreshing agents, anti-inflammatory agents, skin brightening agents (such as whitening agents, cell activators, skin roughness improvement agents, blood circulation promoters, skin astringents, and anti-seborrheic agents), vitamins, amino acids, nucleic acids, hormones, inclusion compounds, bioactive substances, medicament active components, perfumes, and the like, by selecting a method or quantitative range similar to those disclosed in (International Patent Publication WO2017/191798).

In particular, the silicone elastomer particles of the present invention have superior feel during use, texture, handling workability, storage stability, dispersibility, and high oil absorption properties that are equal or superior to conventionally known silicone particles, silicone composite particles coated with silsesquioxane, and silicone particles containing an oil agent. Therefore, a particularly preferred appearance, feel during use, and the like can be achieved in the following:
(1) Cosmetic material compositions and formulations containing oil-based media such as oil agents and the like (oil-based cosmetic raw materials);
(2) Cosmetic material compositions and formulations containing lipophilic ultraviolet light blocking components (such as octyl paramethoxycinnamate and the like); and
(3) Cosmetic material compositions and formulations containing inorganic powders such as colorants, pigments, or the like. These specific formulations are further described in detail in the Examples.

In addition thereto, the silicone elastomer particles of the present invention can be easily designed for aqueous dispersions. Therefore, in aqueous cosmetic material compositions and formulations, the silicone elastomer particles provide excellent formulation design freedom and blending stability, and thus can achieve a suitable feel during use. These specific formulations are further described in detail in the Examples.

The cosmetic material of the present invention can be easily manufactured by simply uniformly mixing the cosmetic product raw material of the present invention and other cosmetic product raw materials as described above. As mixing means, various mixing and kneading devices normally used in the manufacture of cosmetic products can be used. Examples of such devices include a homomixer, a paddle mixer, a Henschel mixer, a homodisper, a colloidal mixer, a propeller agitator, a homogenizer, an in-line continuous emulsifier, an ultrasonic emulsifier, and a vacuum kneading machine.

### [Organic Resin Additives and Organic Resins, Paints, and Coating Agents]

The silicone elastomer particles of the present invention are also extremely useful as an organic resin additive due to the properties described above. Specifically, the silicone elastomer particles of the present invention have superior uniform dispersibility in organic resins and, if desired, excellent stress relief properties, and the like. In addition, the particles have very excellent handling workability and storage stability because aggregation is less likely to occur even after long-term storage. Furthermore, a member, paint film, or coating film obtained by curing an organic resin containing the silicone elastomer particles has improved flexibility (including the softness of a coating layer), durability, and adhesion to and followability of the substrate, is particularly pliable, and has superior thermal shock resistance. Therefore, it is extremely useful as a highly functional organic resin, paint, or coating agent for use in electronic materials.

### [Organic Resin]

A curable organic resin composition or a thermoplastic resin is suitably exemplified as an organic resin containing the silicone elastomer particles of the present invention. Of these, curable resins are suitable for electronic materials such as semiconductor substrates and the like. More specifically, examples of the curable organic resin composition include, phenolic resin, formaldehyde resin, xylene resin, xylene-formaldehyde resin, ketone-formaldehyde resin, furan resin, urea resin, imide resin, melamine resin, alkyd resin, unsaturated polyester resin, aniline resin, sulfone-amide resin, silicone resin, epoxy resin, and copolymer resins of these resins, and two or more of these curable resins can be combined. In particular, the curing resin is preferably at least one type selected from the group consisting of an epoxy resin, a phenolic resin, an imide resin, and a silicone resin. The epoxy resin can be any compound containing glycidyl or alicyclic epoxy groups, and examples include o-cresol novolac epoxy resins, phenol novolac epoxy resins, biphenyl epoxy resins, bisphenol A epoxy resins, bisphenol F epoxy resins, dicyclopentadiene epoxy resins, naphthalene epoxy resins, anthracene epoxy resins, naphthol aralkyl epoxy resins, polyvinylphenol epoxy resins, diphenylmethane epoxy resins, diphenylsulfone epoxy resins, triphenolalkane epoxy resins, cresol-naphthol co-condensation epoxy resins, bisphenylethylene epoxy resins, fluorene epoxy resins, stilbene epoxy resins, spiro-coumarone epoxy resins, norbornene epoxy resins, terpene epoxy resins, phenolcyclohexane epoxy resins, halogenated epoxy resins, imide-group-containing epoxy resins, maleimide-group-containing epoxy resins, allyl group-modified epoxy resins, and silicone-modified epoxy resins. Examples of this phenolic resin include a polyvinylphenol type, a phenol novolac type, a naphthol type, a terpene type, a phenol dicyclopentadiene type, a phenol aralkyl type, a naphthol aralkyl type, a triphenol alkane type, a dicyclopentadiene type, a cresol naphthol co-condensation type, and a xylene-naphthol co-condensation type. An example of a silicone resin is an epoxy-modified silicone resin generated by a reaction between an epoxy resin and a silanol group or a silicon-bonded alkoxy group in the silicone resin. Examples of the curing mechanisms of such curable resins are thermal curing, high energy beam curing such as ultraviolet light, radiation, and the like, moisture curing, condensation reaction curing, and addition reaction curing. The properties of such curable resins at 25 °C are not limited, and may be in either a liquid state or a solid state that softens upon heating.

Another optional component such as a curing agent, curing promoter, filler, photosensitizer, higher fatty acid metal salt, ester wax, plasticizer, or the like can be added to the organic resin containing the silicone elastomer particles of the present invention. Examples of curing agents include: organic acids such as carboxylic acids, sulfonic acids, and the like and anhydrides thereof; organic hydroxy compounds; organic silicon compounds having a silanol group, an alkoxy group, or a halogeno group; and primary or secondary amino compounds, and two or more types can be combined. Examples of the curing promoter include: tertiary amine compounds, organic metal compounds such as aluminum, zirconium, and the like; organophosphorus compounds such as phosphine and the like; other heterocyclic amine compounds, boron complex compounds, organic ammonium salts, organic sulfonium salts, organic peroxides, and catalysts for hydrosilylation. Examples of these fillers include: fibrous fillers such as glass fiber, asbestos, alumina fiber, ceramic fiber composed of alumina and silica, boron fiber, zirconia fiber, silicon carbide fiber, metal fiber, polyester fiber, aramid fiber, nylon fiber, phenolic fiber, natural animal and plant fiber, and the like; and powdered fillers such as fused silica, precipitated silica, fumed silica, calcined silica, zinc oxide, calcined clay, carbon black, glass beads, alumina, talc, calcium carbonate, clay, aluminum hydroxide, barium sulfate, titanium dioxide, aluminum nitride, silicon carbide, magnesium oxide, beryllium oxide, Kaolin, mica, zirconia, and the like. Two or more of these can be combined. In the case of epoxy resins, it is particularly preferable to include an amine curing agent.

The silicone elastomer particles of the present invention may be added as an additive to a thermoplastic resin other than those described above, and may be used as a modifier of physical properties such as surface lubricants, stress relief agents, and the like, or modifiers of optical properties such as light scattering agents and the like. The type of thermoplastic resin is not particularly limited, and may be at least one polymer selected from a group consisting of: polycarbonate resins; polyester resins; polyether resins; polylactic acid resins; polyethylene, polypropylene, ethylene-propylene copolymers, and other polyolefin resins; polystyrene resins; styrene copolymers; tetrafluoroethylene and other fluorine-based polymers; polyvinyl ethers; and cellulose-based polymers, or a composite resin containing a combination of these. The silicone resin coated silicone elastomer particles of the present invention can be uniformly dispersed in these thermoplastic resins (including master batches) using a mixing device such as a biaxial or single-axis extruder, a kneader mixer, or the like, and may be molded into a desired shape, such as a film form or the like, for use.

The added amount of the silicone elastomer particles of the present invention may be selected as appropriate according to the physical properties required of the organic resin, but is generally within a range of 0.1 to 30 parts by mass with respect to 100 parts by mass of the organic resin, and may be within a range of 0.5 to 10 parts by mass. The reason is that if the amount of the particles added is less than the lower limit, the performance such as stress relief properties for the resin or the like may become insufficient, and the pliability and thermal shock resistance of the resulting cured organic resin product may decrease, and in particular, the thermal shock resistance tends to decrease after moisture absorption. On the other hand, if the amount exceeds the upper limit, the organic resin or the paint/coating agent after blending may become thickened and the handling workability may decrease, and the mechanical properties of the resulting organic resin cured product tend to decrease.

Furthermore, the silicone elastomer particles of the present invention are superior in stress relief when added to an organic resin, and thus may be added to an epoxy resin or the like for printed wiring boards to form a prepreg. Furthermore, a copper foil containing filler particles for a printed wiring board provided with a resin layer containing the silicone elastomer particles of the present invention on one side of the copper foil may be formed and used for a copper clad laminate (CCL) application.

### [Paints, Coating Agents]

Examples of paints and coating agents containing the silicone elastomer particles of the present invention include ambient temperature curing types, ambient temperature drying types, and heat curing types, with examples based on the properties thereof including aqueous types, oil-based types, and powdered types, and examples based on the vehicle resin including polyurethane resin paint, butyral resin paint, long oil phthalate resin paint, alkyd resin paint, amino alkyd resin paint made up of amino resin and alkyd resin, epoxy resin paint, acrylic resin paint, phenol resin paint, silicone modified epoxy resin paint, silicone modified polyester resin paint, and silicone resin paint.

The added amount of the silicone elastomer particles of the present invention can be selected as appropriate according to the physical properties required for the paint/coating agent, but in order to impart uniform and soft matting properties to the resulting coating film, the added amount is preferably within a range of 0.1 to 150 parts by mass with respect to 100 mass parts of solid content of the paint, more preferably within a range of 0.1 to 100 parts by mass, even more preferably 0.1 to 50 parts by mass, and particularly preferably 0.1 to 20 parts by mass. If the amount of the particles added is less than the lower limit, performance such as matting, adhesion, stress relief properties, and the like of the coating film may be insufficient. If the amount of the particles exceeds the upper limit, the organic resin and the paint/coating agent after blending may become thickened and the handling workability may decrease.

The paints and coating agents containing the silicone elastomer particles of the present invention may contain: alcohols such as methanol, ethanol, and the like; ketones such as methyl ethyl ketone, methyl isobutyl ketone, and the like; esters such as ethyl acetate, butyl acetate, cellosolve acetate, and the like; amides such as N,N-dimethylformamide and the like; olefins such as hexane, heptane, octane, and the like; organic solvents such as toluene, xylene, and other aromatic hydrocarbons; known inorganic fillers such as reinforcing silica and the like; organic fillers; curing promoters; silane coupling agents; carbon black and other pigments; dyes; antioxidants; thickeners containing a high molecular weight compound; flame retardants; and weather resistance imparting agents.

### [As Eco-Friendly Material]

As described above, unlike conventional non-biodegradable thermoplastic resin particles and silicone particle materials, the silicone elastomer particles of the present invention are expected to be biodegradable in a biodegradable environment, where crosslinked structures formed between silicon atoms within the silicone elastomer particles are at least partially cleaved, and primary particles of the silicone elastomer particles have a property of disintegrating with the generation of a polyorganosiloxane with a non-crosslinked structure. Therefore, use is possible as an "eco-friendly" cosmetic raw material with low environmental burden and environmental risk that complies with regulations on microplastics and the like, and is expected to appeal as a biodegradable and "eco-friendly" material to users and consumers who are concerned about global environmental impact.

### EXAMPLES

With the reactive group-containing polycaprolactone compound according to the present invention (specifically, a compound selected from the aforementioned (meth)acryl-modified polycaprolactone compound and alkenyl-modified polycaprolactone compound), silicone elastomer particles using the compound as a raw material, and a method for producing the particles will be described in detail with reference to Examples and Comparative Examples. However, the present invention is not limited only to these examples. Viscosity in the examples is the value at 25°C. The properties of each silicone particle were measured as follows. Note that in the examples and the like, unless otherwise specified, silicone particles is a generic term for particles made of a silicone cured product (cured silicone particles), and does not include emulsions.

### [Average Primary Particle Diameter of Emulsion Particles]

The emulsion before the addition of the radical polymerization initiator or before the addition of the hydrosilylation catalyst was measured using a laser diffraction particle diameter distribution analyzer (LS-230 from Beckman Coulter), and the median diameter (particle diameter corresponding to 50% of the cumulative distribution, 50% particle diameter) was used as the average particle diameter.

### [Average Secondary Particle Diameter of Silicone Particles (Powder)]

Using ethanol as a dispersion medium, the particle diameter of the cured silicone particles was measured with a laser diffraction particle diameter distribution analyzer (Mastersizer 3000 from Malvern Panalytical), and the median diameter of the cured silicone particles in ethanol (particle diameter corresponding to 50% of the cumulative distribution, D90, µm) and the arithmetic dispersion (particle diameter distribution SD, µm2) values were obtained. For the measurement sample, cured silicone particles (1 g) and ethanol (100 mL) were dispersed in a 300 mL cup using stirring blades and an ultrasonic vibrator.

Component (A) used in the examples and comparative examples is as follows. Note that "Me" refers to "methyl group".
(a2-1) Dimethylsiloxane/methylhydrogensiloxane copolymer capped at both molecular terminals with a trimethylsiloxy group, expressed by structural formula Me₃SiO(Me₂SiO)₃₄-(Me(H)SiO)₁₆SiMe₃
(a1-1) Methacryl-modified silicone polymer (viscosity 1524 mPas at 25°C) expressed by the following structural formula: 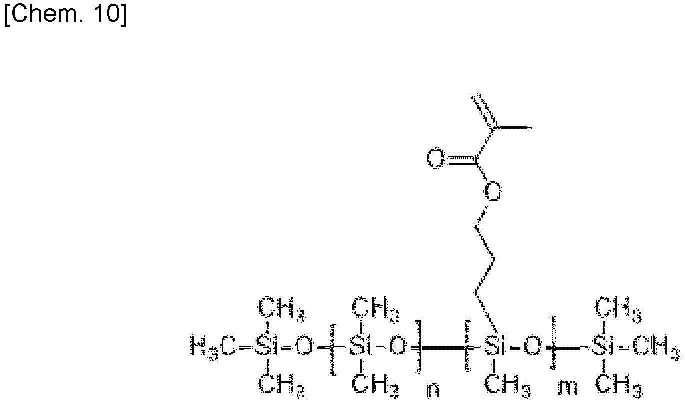 (m=4, n=267 in the formula). A synthesis example is provided below.

### [Synthesis Example]

92.07 parts by weight of dodecamethylcyclosiloxane and 0.01 parts by weight of MEHQ (hydroquinone monomethyl ether, polymerization inhibitor), and 5.88 parts by weight of 3-methacryloxypropylmethyldimethoxysilane were placed in a 4-neck separable flask. The mixture was heated while stirring at 200 rpm and blowing N₂, and when the temperature reached 50°C, 0.05 parts by weight of trifluoromethanesulfonic acid and 1.09 parts by weight of water were added. After reacting at 55°C for 2 hours, the liquid temperature was heated to 70°C. Furthermore, the pressure was reduced to 100 mmHg to remove the methanol produced as a by-product for about 1 hour. Thereafter, 0.90 parts by weight of hexamethyldisiloxane was added and the reaction was carried out for 3 hours. After the reaction, ammonia gas was bubbled to neutralize the trifluoromethanesulfonic acid, and the generated salt was removed by filtration using diatomaceous earth. The filtrate was subjected to reduced pressure processing at 150°C for 3 hours to remove volatile components. According to C, Si-NMR analysis, (a1-1) a methacryl-modified silicone polymer was obtained, having 267 units of dimethylsiloxane and 4 units of methacryl group-introduced siloxane, with a viscosity of 1524 mPas.

### [Examples 1 to 6: Synthesis of (meth)acryl-modified polycaprolactone compounds Nos. 1 to 3 and alkenyl-modified polycaprolactone compounds Nos. 4 to 6]

Hereinafter, a synthesis example of a (meth)acryl-modified polycaprolactone compound according to an embodiment of the present invention using a reaction between a polycaprolactone compound having a polyol terminal structure and a specific (meth)acryloyl chloride compound will be described. These components are components used as silicone elastomer raw materials in Example 5 and subsequent examples described below.

### [Example 1 : (meth)acryl-modified polycaprolactone compound No. 1]

A four-neck separable flask was filled with 18.81 parts by weight of Placcel 205 (trade name, manufactured by DAICEL CORPORATION, diol type polycaprolactone, mol. wt.: 530), 18.81 parts by weight of chloroform, 8.77 parts by weight of triethyl amine and 0.03 parts by weight of MEHQ (hydroquinonemonomethyl ether, polymerization inhibitor). 6.55 parts by weight of acryloyl chloride was added dropwise while stirring at 200 rpm and aerating with N₂ containing 2% O₂. The reaction was carried out while cooling with a water bath so that the exothermic temperature did not exceed 30°C. After completion of the dropwise addition, the stirring time was extended by 1 hour. Furthermore, the liquid temperature was raised to 50°C, and the mixture was aged for about 2 hours. Next, 28.21 parts by weight of water-1 was added and stirred well. Thereafter, the mixture was transferred to a separatory funnel, and the bottom phase containing the modified polycaprolactone was removed. Furthermore, 18.81 parts by weight of water-2 was added to make the mixture uniform. The sample was again transferred to a separatory funnel and left overnight for further separation. Chloroform was removed under reduced pressure while bubbling with N₂ containing 2% O₂ to obtain a transparent orange polymer. Based on H-NMR analysis, a peak derived from an acrylic group appeared in the polymer, and it was confirmed that the polymer was polycaprolactone having a terminal modified with an acrylic group ((meth)acryl-modified polycaprolactone compound No. 1) having the following structure. 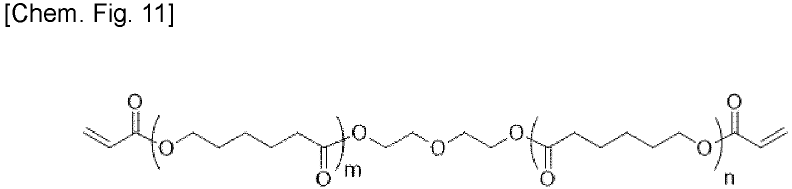 (Where m + n = 3.7.)

### [Example 2: (meth)acryl-modified polycaprolactone compound No. 2]

An acrylic group-modified polycaprolactone ((meth)acryl-modified polycaprolactone compound No. 2) having the following structure was obtained in the same manner as in Example 1 except that 17.92 parts by weight of Placcel305 (triol type polycaprolactone manufactured by DAICEL CORPORATION, molecular weight: 550), 17.92 parts by weight of chloroform, 10.27 parts by weight of triethyl amine, 0.05 parts by weight of MEHQ, 9.02 parts by weight of acryloyl chloride, 26.89 parts by weight of water-1, and 17.92 parts by weight of water-2 were used. 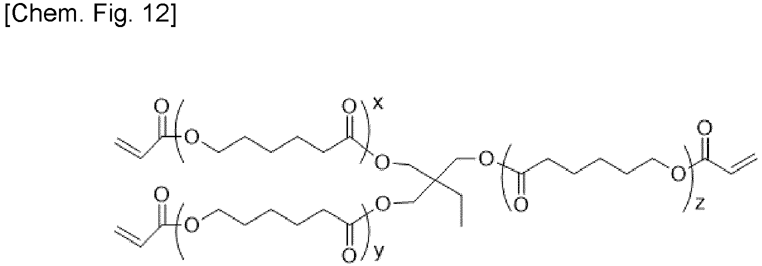 (Where x + y + z = 3.7.)

### [Example 3 : (meth)acryl-modified polycaprolactone compound No. 3]

An acrylic group-modified polycaprolactone ((meth)acryl-modified polycaprolactone compound No. 2) having the following structure was obtained in the same manner as in Example 1 except that 18.70 parts by weight of Placcel410 (tetraol type polycaprolactone manufactured by DAICEL CORPORATION, molecular weight: 1000), 18.70 parts by weight of chloroform, 7.86 parts by weight of triethyl amine, 0.02 parts by weight of MEHQ, 6.91 parts by weight of acryloyl chloride, 28.69 parts by weight of water-1, and 19.12 parts by weight of water-2 were used. 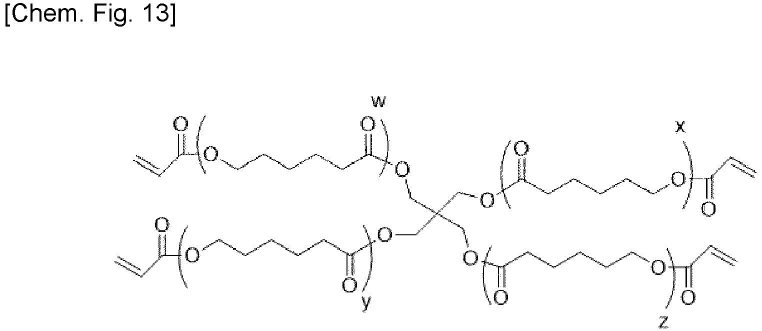 (wherein w + x + y + z = 7.6.)

### [Example 4: Alkenyl-modified polycaprolactone compound No. 4]

27.36 parts by weight of Placcel305 (trade name, manufactured by DAICEL CORPORATION, triol type polycaprolactone, molecular weight: 550), 27.36 parts by weight of chloroform, and 14.41 parts by weight of potassium carbonate were placed in a 4-neck separable flask. While stirring at 200 rpm and aerating with N₂, 30.86 parts by weight of undecenoyl chloride was added dropwise. The reaction was carried out while cooling with a water bath so that the exothermic temperature did not exceed 30°C. After dripping was completed, heating and stirring were performed for 24 hours under a nitrogen atmosphere to obtain a reaction product. The organic portion was transferred to another four-neck flask, and while bubbling with N₂, chloroform and excess undecylenic acid were removed under reduced pressure to obtain a transparent polymer. Based on H-NMR analysis, a peak derived from an undecyl group appeared in the polymer, so the polymer was polycaprolactone having a terminal modified with an undecyl group (alkanyl-modified polycaprolactone compound No. 4) having the following structure. 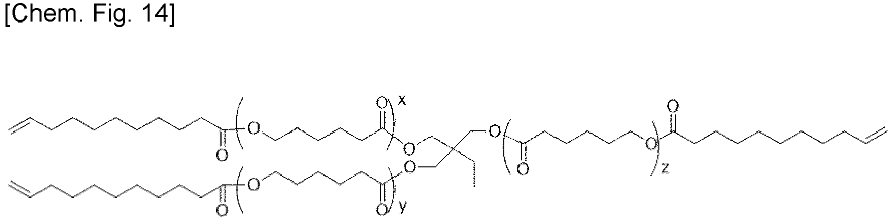 (Where x + y + z = 3.7.)

### [Example 5: Alkenyl-modified polycaprolactone compound No. 5]

57.62 parts by weight of Placcel 210 (diol-type polycaprolactone, molecular weight: 1000, manufactured by Daicel Corporation) and 42.35 parts by weight of vinylsilazane were added to a four-neck separable flask. 0.02 parts by weight of trifluoromethanesulfonic acid was added dropwise while the mixture was heated to 50°C with stirring and N₂ aeration. The reaction was carried out at 50°C for 4 hours. 3.00 parts by weight of KYOWAD 500 (synthetic hydrotalcite manufactured by Kyowa Chemical Industry Co., Ltd.) was added and stirred for 1 hour. After filtration, byproducts were removed while performing N₂ bubbling to obtain a transparent polymer. Based on H-NMR and Si-NMR analysis, a peak derived from a vinylsiloxy group appeared in the polymer, so the polymer was polycaprolactone having a terminal modified with an vinylsiloxy group (alkanyl-modified polycaprolactone compound No. 5) having the following structure. 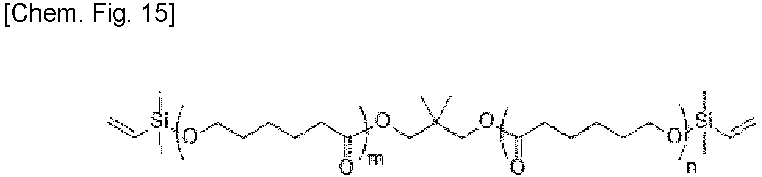 (Where m + n = 7.9).

### [Example 6: Alkenyl-modified polycaprolactone compound No. 6]

A four separable flask was charged with 35.12 parts by weight of Pluronic L-31 (trade name, manufactured by Adeka Corporation, diol type polyoxyethylene-polyoxypropylene copolymer, molecular weight: about 1100), 49.70 parts by weight of chloroform, and 0.22 parts by weight of triazabicyclodecene. 14.58 parts by weight of ε-caprolactone was added dropwise with aeration of N₂. Stirring was performed at room temperature for 4 hours. After the reaction, 0.38 parts by weight of benzoic acid was added. After allowing to stand overnight, chloroform was removed by heating under reduced pressure with N₂ bubbling to obtain a transparent polymer. 83.21 parts by weight of the obtained polymer and 16.79 parts by weight of vinylsilazane were added. The mixture was heated to 50°C under N₂ bubbling, 0.02 parts by weight of trifluoromethanesulfonic acid was added, and the mixture was reacted for 4 hours. After filtration, the by-components were removed by N2 bubbling to obtain a transparent polymer. Based on H-NMR and Si-NMR analysis, a peak derived from a vinylsiloxy group appeared in the polymer, so the polymer was polycaprolactone having a terminal modified with an vinylsiloxy group (alkanyl-modified polycaprolactone compound No. 6) having the following structure. 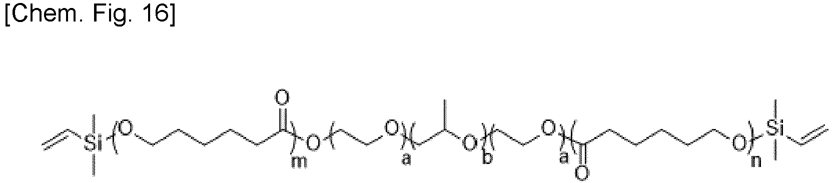 (where m + n=4.0 and a + b=18)

### [Examples 7 to 12, Comparative Example 1: Production of silicone elastomer particles]

Examples 7 to 12 show production examples of silicone elastomer particles obtained by using the (meth)acryl-modified polycaprolactone compound as a raw material. Comparative Example 1 is non-silicone-based polymer particle obtained using only a (meth)acrylic-modified polycaprolactone compound as a raw material.

### [Example 7: Silicone elastomer particle No. 1 (hydrosilylation reaction type)]

The organohydrogenpolysiloxane of component (a2-1) and (b4) alkenyl-modified polycaprolactone compound No. 4 were uniformly mixed at a mass ratio of 37: 63 at room temperature. Thereafter, this composition was dispersed in an aqueous solution at 25°C that was composed of 30 parts by mass of purified water and 0.5 parts by mass of polyoxyethylene alkyl (C12-14) ether.

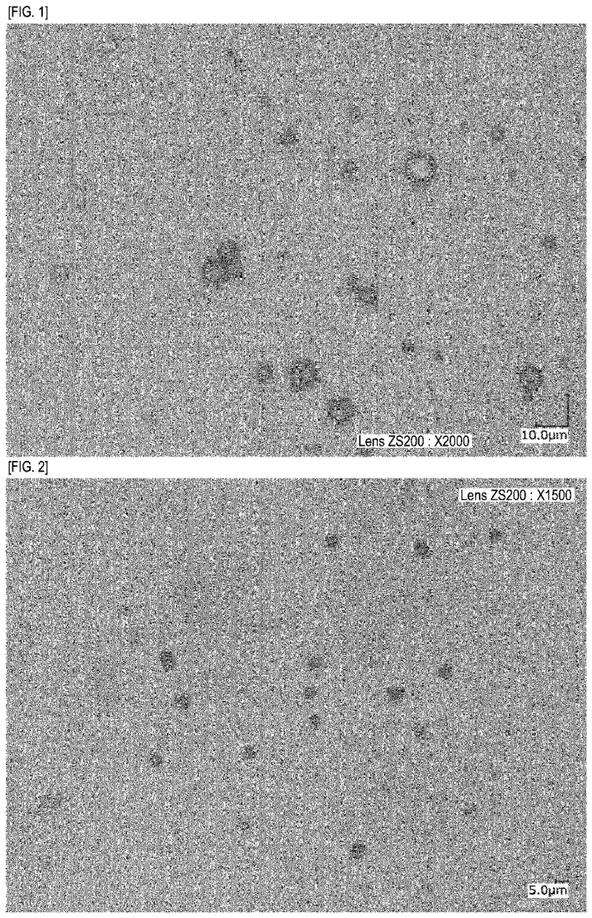

After the mixture was uniformly emulsified using a colloid mill, 526 parts by mass of purified water was added for dilution to prepare the emulsion. Next, an isopropyl alcohol solution of platinum chloride (an amount of which platinum metal set to 10 ppm by mass in this composition) was added to the emulsion as an aqueous dispersion with polyoxyethylene alkyl (C12-14) ether and pure water, and the emulsion was stirred. Subsequently, the silicone rubber was let stand at 50 °C for 4 hours to prepare a uniform aqueous suspension of elastomer particles. The aqueous suspension was then filtered and the residue was dried in an oven at 50°C for five hours to obtain silicone elastomer particle No. 1. The average primary and secondary particle diameter of the resulting elastomer particles was 4.03 µm and 15.3 µm, respectively. Note that the electron micrograph is depicted in FIG. 1.

### [Example 8: Silicone elastomer particles No. 2 (radical polymerization type)]

The methacryl-modified silicone polymer (a1-1) and (b1) (meth)acryl-modified polycaprolactone compound No. 1 were uniformly mixed at a mass ratio of 30:70 at room temperature, and olive oil (manufactured by Summit Oil Co., Ltd.) was also added in an amount corresponding to 20% of the total composition, followed by mixing. Next, this composition was dispersed in an aqueous solution at 25°C containing 0.27 parts by mass of GOHSENOL EG-05C and 0.53 parts by mass of GOHSENOL EG-18P in 46 parts by mass of pure water, and after uniform emulsification using a colloid mill, 300 parts by mass of pure water was added. After heating in a 1 L flask to 60°C, an aqueous solution of 0.5 g potassium persulfate (manufactured by Sigma-Aldrich) dissolved in 9.5 g water was added dropwise over one minute. The emulsion was stirred at 60°C for three hours at 100 rpm to perform radical polymerization to prepare a uniform aqueous suspension of silicone rubber particles. The aqueous suspension was then filtered and washed with 200 mL of ethanol and 100 mL of acetone. The residue was dried in an oven at 70°C for 3 hours to obtain silicone elastomer particles No. 2. The average primary and secondary particle diameters of the resulting silicone elastomer particles were 3.41 µm and 31.1 µm.

### [Example 9: Silicone elastomer particles No. 3 (radical polymerization type)]

Component (a1-1), methacrylic modified silicone polymer, and (b2) (meth)acrylic modified polycaprolactone compound No. 2 were uniformly mixed at a mass ratio of 30:70 at room temperature. Otherwise, silicone elastomer particles No. 3 were obtained in the same manner as in Example 8. The average primary and secondary particle diameters of the resulting silicone elastomer particles were 3.43 µm and 31.1 µm. Note that the electron micrograph is depicted in FIG. 2.

### [Example 10: Silicone elastomer particles No. 4 (radical polymerization type)]

Component (a2-1), methacrylic modified silicone polymer, and (b3) (meth)acrylic modified polycaprolactone compound No. 3 were uniformly mixed at a mass ratio of 30:70 at room temperature. Otherwise, the silicone elastomer particles were obtained in the same manner as in Example 8. The average primary and secondary particle diameters of the resulting silicone elastomer particles were 4.42 µm and 336 µm.

### [Example 11: Silicone elastomer particle No. 5 (hydrosilylation reaction type)]

The organohydrogenpolysiloxane of component (a1-1) and (b5) alkenyl-modified polycaprolactone compound No. 5 were uniformly mixed at a mass ratio of 18.7: 81.3 at room temperature. Otherwise, silicone elastomer particles No. 5 were obtained in the same manner as in Example 7. The average primary and secondary particle diameters of the resulting silicone elastomer particles were 3.73 µm and 18.5 µm.

### [Example 12: Silicone elastomer particle No. 6 (hydrosilylation reaction type)]

The organohydrogenpolysiloxane of component (a1-1) and (b6) alkenyl-modified polycaprolactone compound No. 6 were uniformly mixed at a mass ratio of 15:85 at room temperature. Otherwise, silicone elastomer particles No. 6 were obtained in the same manner as in Example 7. The average primary and secondary particle diameters of the resulting silicone elastomer particles were 0.95 µm and 30.2 µm.

### [Comparative Example 1 (radical polymerization type)]

Non-silicone polymer particles were obtained in the same manner as in Example 6, except that a polyorganosiloxane component was not used and 100 mass parts of only (b1) (meth)acryl-modified polycaprolactone compound No. 1 was used. The average primary and secondary particle diameters of the resulting particles were 2.91 µm and 70.0 µm.

The average primary and secondary particle diameters of each particle obtained from the above Examples 7 to 12 and Comparative Example 1 are summarized in Table 1 below. Note that as depicted in FIG. 1 and FIG. 2, digital microscope observation of the silicone elastomer particles obtained in Examples 7 and 9 confirmed that the particles were essentially free of aggregation and dispersibility was excellent.

**[Table 1]**

| | Average primary particle diameter (µm) | Average secondary particle diameter (µm) |
|---|---|---|
| Silicone elastomer particle No. 1 | 4.03 | 15.3 |
| (Example 7) | | |
| Silicone elastomer particle No. 2 | 3.41 | 31.1 |
| (Example 8) | | |
| Silicone elastomer particle No. 3 | 3.43 | 31.1 |
| (Example 9) | | |
| Silicone elastomer particle No. 4 | 4.42 | 336 |
| (Example 10) | | |
| Silicone elastomer particle No. 5 | 3.73 | 18.5 |
| (Example 11) | | |
| Silicone elastomer particle No. 6 | 0.95 | 30.2 |
| (Example 12) | | |
| Comparative example 1 | 2.91 | 70.0 |

### [Cosmetic Material Formulation Example]

The following are formulation examples of cosmetic materials of the present invention that can contain silicone elastomer particles, which is one aspect of the present invention. However, the present invention is not limited thereto.

### [Examples 9, 10 and Comparative Example 2]

Panelists compared and evaluated the feel during use of loose powder in which the silicone elastomer particles in the compositions listed in the following Table 3 were used.

### Evaluation of texture

The slipperiness of the samples applied to inner forearms of 18 panelists was evaluated using the criteria in Table 2 below.

**[Table 2]**

| Evaluation results | Evaluation Indicators |
|---|---|
| ○ | 12 or more of 18 respondents said the slipperiness was favorable |
| Δ | 7 to 11 of 18 respondents said the slipperiness was favorable |
| × | 6 or fewer of 18 respondents said the slipperiness was favorable |

**[Table 3]**

| Phase | Component | Product name and supplier | Example 13 | Example 14 | Example 15 | Example 16 | Comparative example 2 |
|---|---|---|---|---|---|---|---|
| A | Silicone elastomer particles of Example 7 No. 1 | | 10 | | | | |
| | Silicone elastomer particles of Example 8 No. 2 | | | 10 | | | |
| | Silicone elastomer particles of Example 11 No. 5 | | | | 10 | | |
| | Silicone elastomer particles of Example 12 No. 6 | | | | | 10 | |
| | Elastomer particles of Comparative Example 1 | | | | | | 10 |
| | Bis(hydroxyethylpropyl) dimethicone | DOWSIL^{™} 5562 Carbinol Fluid | 6 | 6 | 6 | 6 | 6 |
| B | Talc | JA-46R produced by ASADA MILLING CO., LTD. | 76 | 76 | 76 | 76 | 76 |
| | Silylated silica | DOWSIL^{™} VM-2270 Aerogel Fine Particle | 2 | 2 | 2 | 2 | 2 |
| | Kaolin | | 1.5 | 1.5 | 1.5 | 1.5 | 1.5 |
| | Titanium oxide | SI Titanium CR-50 available from Miyoshi Kasei, Inc. | 3.92 | 3.92 | 3.92 | 3.92 | 3.92 |
| | Iron oxide yellow | SI-YELLOW-LLXLO available from Miyoshi Kasei, Inc. | 0.46 | 0.46 | 0.46 | 0.46 | 0.46 |
| | Iron oxide red | SA-Bengara Cloisonne available from Miyoshi Kasei, Inc. | 0.09 | 0.09 | 0.09 | 0.09 | 0.09 |
| | Iron oxide black | SA-Black BL-100 available from Miyoshi Kasei, Inc. | 0.02 | 0.02 | 0.02 | 0.02 | 0.02 |
| Evaluation of texture | | | ○ | Δ | ○ | ○ | × |

### (Method of preparation)

1. Phase A is mixed.
2. Phase B is mixed.
3. Phases A and B are stirred until uniform.

As shown in Table 3, loose powder using the silicone elastomer particles of the present invention (Example 1, hydrosilylation reaction type) was evaluated to have favorable slipperiness, unlike loose powders that use other particles (Comparative Example 1).

### [Examples 17,18, and 19, and Comparative Examples 3, 4, and 5]

The panelists compared and evaluated the feel during use of water-in-oil sunscreen agents in which the silicone elastomer particles in the compositions listed in Table 5 were used.

### Evaluation of texture

The 18 panelists evaluated the spreadability and white residue when the samples were applied to inner forearms using the criteria in Table 4 below.

**[Table 4]**

| Evaluation results | Evaluation Indicators |
|---|---|
| ○ | 12 or more of 18 persons responded favorable |
| Δ | 7 to 11 of 18 persons responded favorable |
| × | 6 or fewer of 18 persons responded favorable |

### [SPF value and PA value]

An evaluation target (sunscreen cosmetic composition) was uniformly applied to HELIOPLATE HD6 (available from HelioScreenLab) so as to be 2 mg/cm² to measure the SPF value and PA value using the SPF measuring device UV-1000S (available from Labsphere). The values listed in Table 1 to 3 are average values excluding the maximum value and the minimum value by performing 10 measurements on each of three test samples.

**[Table 5]**

| Phase | Component | | Product name and supplier | | Comparative example 3 | Example 17 | Example 18 | Example 19 | Comparative example 4 | Comparative example 5 |
|---|---|---|---|---|---|---|---|---|---|---|
| A | 50% titanium dioxide dispersion (dimethicone 2cs liquid) | | | | 36 | 36 | 36 | 36 | 36 | 36 |
| | 50% zinc oxide dispersion (dimethicone 2cs liquid) | | | | 20 | 20 | 20 | 20 | 20 | 20 |
| | Caprylyl methicone | | DOWSIL^{™} FZ-3196 | | 10 | 10 | 10 | 10 | 10 | 10 |
| | Dimethicone | | XIAMETER^{™} PMX-200 2cs | | 9.8 | 6.8 | 6.8 | 6.8 | 6.8 | 6.8 |
| | Silicone emulsifiers | | DOWSIL^{™} ES-5300 Formulation Aid | | 2 | 2 | 2 | 2 | 2 | 2 |
| | Silicone elastomer particles of Example 7 No. 1 | | | | | 3 | | | | |
| | Silicone elastomer particles of Example 11 No. 5 | | | | | | 3 | | | |
| | Silicone elastomer particles of Example 12 No. 6 | | | | | | | 3 | | |
| | Elastomer particles of Comparative Example 1 | | | | | | | | 3 | |
| | Silicone elastomer particles | | DOWSIL^{™} 9701 Cosmetic Powder | | | | | | | 3 |
| B | Sodium citrate | | | | 0.2 | 0.2 | 0.2 | 0.2 | 0.2 | 0.2 |
| | Sodium chloride | | | | 0.5 | 0.5 | 0.5 | 0.5 | 0.5 | 0.5 |
| | BG | | | | 3 | 3 | 3 | 3 | 3 | 3 |
| | Water | | | | 17.8 | 17.8 | 17.8 | 17.8 | 17.8 | 17.8 |
| C | Preservative | | Euxyl PE9010 available from Schülke & Mavr | | 0.7 | 0.7 | 0.7 | 0.7 | 0.7 | 0.7 |
| | | | | | | | | | | |
| Evaluation of texture | | | Ease of elongation | | × | ○ | ○ | ○ | Δ | Δ |
| [SPF value and PA value] | | | | | 39 | 109 | 124 | 114 | 80 | 53 |
| | | | | | +++ | ++++ | ++++ | ++++ | ++++ | +++ |

### (Method of preparation)

Mix phase A.
Phase B is mixed.
Phase B is slowly added while stirring Phase A.
Phase C is added to 4 above and then stirred until uniform.

As shown in Table 5, the oil-in-water sunscreen agent using the silicone elastomer particles No. 1 of the present invention (Example 5) was evaluated to have favorable spreadability, a smooth and moist feel, and there was no squeaky feel, unlike Comparative Example 5, which did not contain elastomer particles, as well as sunscreen using other particles (Comparative Example 4). Furthermore, it was found to provide a better feel as compared to the existing product (Example 5). In addition, in Example 11 containing the silicone elastomer particles of the present invention, the SPF value and the PA value could be remarkably improved without impairing the feel during use, as compared with sunscreens containing other particles.

### [Examples 20, 21 and Comparative Examples 6, 7, 8]

The panelists compared and evaluated the feel during use of water-in-oil foundation in which the silicone elastomer particles in the compositions listed in Table 6 were used.

**[Table 6]**

| Phase | Component | Product name and supplier | Comparative example 6 | Comparative example 7 | Example 20 | Example 21 | Comparative example 8 |
|---|---|---|---|---|---|---|---|
| A | Dimethicone | XIAMETER^{™} PMX-200 2cs | 8.55 | 5.55 | 5.55 | 5.55 | 5.55 |
| | Silicone emulsifiers | DOWSIL^{™} ES-5612 Formulation Aid | 4 | 4 | 4 | 4 | 4 |
| | Dimethicone crosspolymer | DOWSIL^{™} EP-9610 Cosmetic Powder | | 3 | | | |
| | Silicone elastomer particles of Example 11 No. 5 | | | | 3 | | |
| | Silicone elastomer particles of Example 12 No. 6 | | | | | 3 | |
| | Elastomer particles of Comparative Example 1 | | | | | | 3 |
| B | Titanium oxide | SI Titanium CR-50 available from Miyoshi Kasei, Inc. | 10.5 | 10.5 | 10.5 | 10.5 | 10.5 |
| | Iron oxide yellow | SI-YELLOW-LLXLO available from Miyoshi Kasei, Inc. | 1.23 | 1.23 | 1.23 | 1.23 | 1.23 |
| | Iron oxide red | SA-Bengara Cloisonne available from Miyoshi Kasei, Inc. | 0.25 | 0.25 | 0.25 | 0.25 | 0.25 |
| | Iron oxide black | SA-Black BL-100 available from Mivoshi Kasei, Inc. | 0.06 | 0.06 | 0.06 | 0.06 | 0.06 |
| | Dimethicone | XIAMETERTM PMX-200 5cs | 5.45 | 5.45 | 5.45 | 5.45 | 5.45 |
| C | Sodium chloride | | 1 | 1 | 1 | 1 | 1 |
| | Glycerin | | 5 | 5 | 5 | 5 | 5 |
| | Water | | Residual | Residual | Residual | Residual | Residual |
| D | Preservative | Euxyl PE9010 available from Schülke & Mavr | 0.7 | 0.7 | 0.7 | 0.7 | 0.7 |
| Evaluation of texture | | | × | Poor emulsificatio n | ○ | ○ | × |

### (Method of preparation)

Phase A is mixed.
Phase B is mixed.
Mix phase A and phase B.
Phase C is slowly added while stirring phases A and B.
Phase D is added to 4 above and then stirred until uniform.

### Evaluation of texture

The 18 panelists evaluated the spreadability and moist feel when the samples were applied to the inner forearm.

**[Table 7]**

| Evaluation results | Evaluation Indicators |
|---|---|
| ○ | 12 or more of 18 persons responded that spreadability was favorable and there was a moist feel after drying |
| Δ | 7 to 11 of 18 persons responded that spreadability was favorable and there was a moist feel after drying |
| × | 6 or fewer of 18 persons responded that spreadability was favorable and there was a moist feel after drying |

### [Examples 22, 23 and Comparative Example 9]

The performance of anti-wrinkle creams with silicone elastomer particles was evaluated by comparing their performance with the compositions listed below in the table.

**[Table 8]**

| | Component | Product name and supplier | Exampl e 22 | Exampl e 23 | Compar ative example 9 |
|---|---|---|---|---|---|
| A | Silicone emulsifier premix | ACULYN^{™} Siltouch Rheology Modifier | 2 | 2 | 2 |
| | Cyclopentasiloxane | DOWSIL^{™} SH245 Fluid | 20 | 20 | 20 |
| | Dimethicone crosspolymer | DOWSIL^{™} EP-9610 Cosmetic Powder | | | |
| | Silicone elastomer particles of Example 11 No. 5 | | 10 | | |
| | Silicone elastomer particles of Example 12 No. 6 | | | 10 | |
| | Elastomer particles of Comparative Example 1 | | | | 10 |
| B | BG | | 3 | 3 | 3 |
| | Methoxyhydroxyphenylisopropylnitrone | AgeCap^{™} Smooth Cosmetic Ingredient | 0.1 | 0.1 | 0.1 |
| C | Phenoxyethanol | NEOLONE PH100 | 0.8 | 0.8 | 0.8 |
| | PEG/PPG-17/6 copolymer | UCON ^{™} Fluid 75H450 | 3 | 3 | 3 |
| | EDTA-2Na | VERSENE ^{™} Na2 Crystals | 0.05 | 0.05 | 0.05 |
| | Water | | Residual | Residual | Residual |
| Soft focus evaluation | | | ○ | ○ | × |

### (Method of preparation)

Phase A is mixed.
Phase B is mixed.
Mix phase B and phase C.
Phase BC is slowly added while stirring Phase A.

### Evaluation of texture

The sample was uniformly applied on a slide glass to a thickness of 75 µm, and the appearance of the characters printed on a paper placed under the slide glass was visually observed.

**[Table 9]**

| Evaluation results | Evaluation Indicators |
|---|---|
| ○ | The characters appear blurred. |
| × | The characters are clearly visible. |

### Enzymatic degradation test of silicone elastomer particles

### [Examples 7, 8, 12 (silicone elastomer particles No. 1, 2, 6) and existing silicone elastomer product]

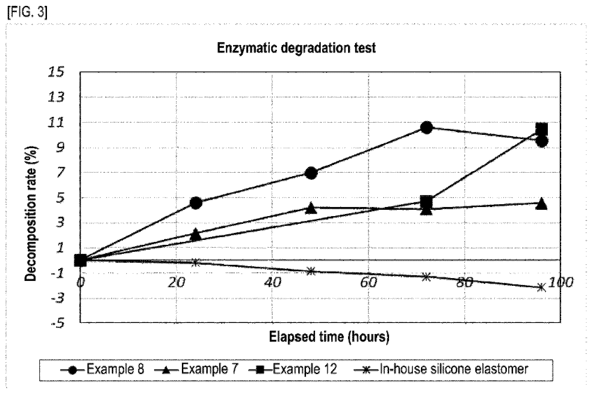

The prepared silicone elastomer particles and an existing silicone elastomer product (Dow Toray Industries, product name: EP-9610 Cosmetic Powder; obtained by hydrosilylation of alkenyl modified polysiloxane and organohydrogen polysiloxane, silicone elastomer particles) were each measured out to 0.1 g and placed in an Eppendorf tube. Type XIII lipase derived from bacteria belonging to the genus Pseudomonas was mixed with 0.1 M phosphate buffered saline (pH 7.4) to prepare an 8 U/mL enzyme solution. 1 mL of the obtained enzyme solution was added to each tube to prepare a test sample. The test samples were placed in an oven at 37°C and tested for up to 96 hours with enzyme exchanges every 24 hours. After the specified time, each sample was removed, washed with water, dried overnight and then oven dried in a vacuum oven. The weight of the sample after absolute drying was measured, and the weight reduction rate was defined as the decomposition rate. The enzymatic degradation test (test time-percent degradation) is shown in FIG. 3.

Silicone elastomer particles No. 1 and 6 of Examples 7 and 12 have a structure crosslinked by a polycaprolactone having an alkenyl group and an organohydrogen polysiloxane having a hydrogen atom bonded to a silicon atom in the presence of a hydrosilylation catalyst, and silicone elastomer particles No. 2 of Example 8 have a structure crosslinked by (meth)acrylic-modified siloxane and polycaprolactone having a (meth)acryl-modified group in the presence of a radical polymerization catalyst.

It is presumed that the silicone elastomer particles according to these examples have biodegradability because the weight thereof decreases over time in the presence of an enzyme. In particular, the silicone elastomer particles according to Examples 8 and 12 both showed a high degradation rate of more than 9% after 96 hours, and the silicone elastomer particles according to the examples of the present application are strongly expected to have biodegradability.

On the other hand, elastomer particles obtained by hydrosilylation of alkenyl-modified siloxane and organohydrogen polysiloxane do not have enzymatically degradable groups like polyesters, and no weight loss was observed in the presence of enzymes, suggesting that they are not biodegradable. In particular, the degradation rates of the silicone elastomer particles in the examples and the existing silicone elastomer product after 96 hours show a clear and significant difference. This strongly suggests that the silicone elastomer particles achieve high biodegradability, which is difficult to achieve with existing silicone elastomer products, in addition to performance and feel advantages comparable to or better than existing products.

## Claims

1. A reactive group-containing polycaprolactone compound having 2 or more modified caprolactone structures expressed by the following structural formula (1): 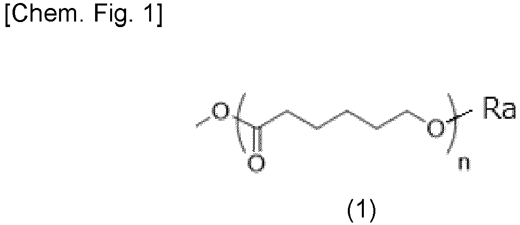 {Where n is a number in the range of 1 to 5, and Ra is a (meth)acryl terminal group or an alkenyl terminal group expressed by - R¹-CR²=CH² (where R¹ is a carbonyl group or a divalent linking group containing one carbonyl group, and R² is a hydrogen atom or a methyl group)}.

2. The reactive group-containing polycaprolactone compound according to claim 1, wherein the compound has 2 to 4 modified polycaprolactone structures expressed by the following structural formula (1) in each molecule and 2 to 20 caprolactone units {-C(=O)-C₅H₁₀-O-}.

3. The reactive group-containing polycaprolactone compound according to claim 1 or claim 2, wherein in the functional group Ra in the following structural formula (1), R¹ represents a carbonyl group or a divalent linking group containing one carbonyl group, selected from organic groups having 1 to 20 carbon atoms or silicon-containing organic groups.

4. The reactive group-containing polycaprolactone compound according to any one of claims 1 to 3, expressed by one or more structural formulas selected from the following structural formulas (1-1) to (1-5): 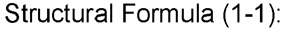 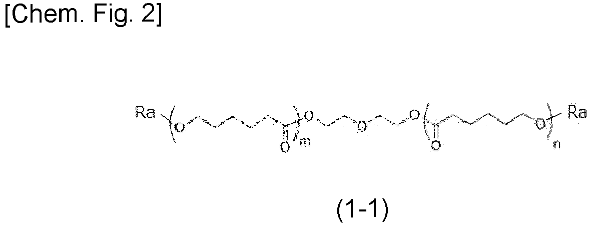 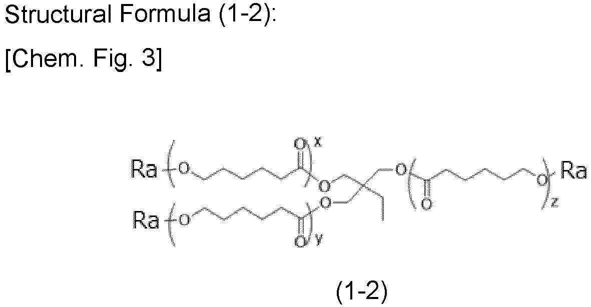 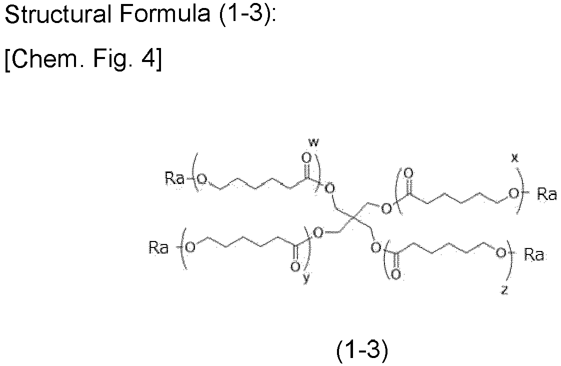 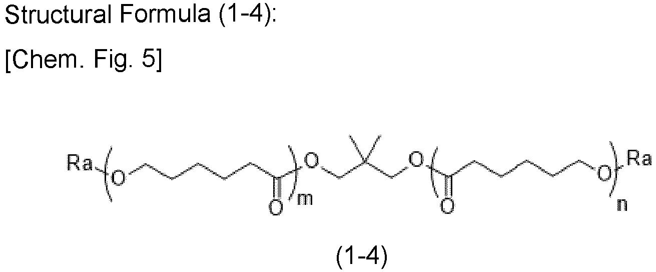 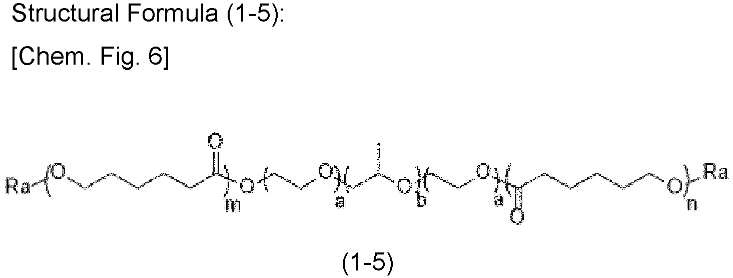 (wherein Ra is the same group as described above; m and n are each independently a number in the range of 1 to 5, and m + n is a number in a range of 2 to 20; w, x, y and z are each independently a number in the range of 1 to 5; x + y + z is a number in a range of 3 to 20; and w + x + y + z is a number in the range of 4 to 20).

5. The reactive group-containing polycaprolactone compound according to any one of claims 1 to 4, which is a synthesis raw material for silicone elastomer particles.

6. A method for producing the reactive group-containing polycaprolactone compound according to any one of claims 1 to 4, comprising:
reacting a polycaprolactone having 2 or more polycaprolactone structures having a polyol terminal structure having in each molecule the following structural formula (1') 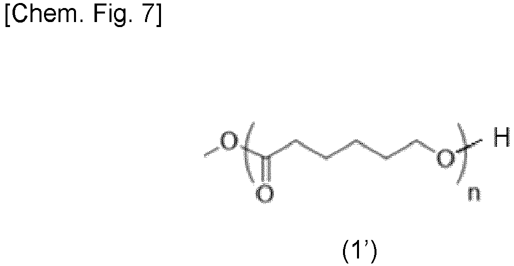
(where n represents a number within a range of 1 to 5)
with a (meth)acryloyl chloride compound expressed by
Cl-C(=O)-R¹-CR²=CH²
(where R¹ is a chemical bond between CH and C(=O) or a divalent organic group with 1 to 20 carbon atoms, and R² is a hydrogen atom or a methyl group)
and an alkenoyl chloride in the presence of a basic catalyst; and
reacting with a tetramethyldivinyldisilazane in the presence of an acid catalyst.

7. A silicone elastomer particle having a structure in which at least two silicon atoms in the silicone elastomer particle are crosslinked by one or more type of reaction selected from a radical polymerization reaction of the reactive group-containing polycaprolactone compound according to any one of claims 1 to 4, and a hydrosilylation reaction with a silicon atom-bonded hydrogen atom.

8. The silicone elastomer particle according to claim 7, further comprising:
a polyorganosiloxane structure expressed by:
-(R¹₂SiO)ₙ-
(where R¹ represents an alkyl group having 1 to 20 carbon atoms, either unsubstituted or substituted with a halogen atom, an aryl group having 6 to 22 carbon atoms, or a hydroxyl group, and n is a number in a range of 1 to 1000)
in the silicone elastomer particle.

9. The silicone elastomer particles according to claim 7 or 8, wherein the silicone elastomer particles include at least:
(A) at least one type of reactive organopolysiloxane selected from the following component (a1) and component (a2);
(a1) an organopolysiloxane having at least three organic groups containing a (meth)acryloxy group of at least one type selected from organic groups containing a methacryloxy group and organic groups containing an acryloxy group in each molecule;
(a2) an organopolysiloxane having at least three silicon atom-bonded hydrogen atoms per molecule;
(B) a reactive group-containing polycaprolactone compound according to any one of claims 1 to 4; and
(C) one or more curing agent selected from radical polymerization initiators and hydrosilylation reaction catalysts; and are obtained by performing in water a crosslinking reaction of crosslinkable silicone emulsified particles obtained by emulsifying in water a crosslinkable silicone composition crosslinkable by one or more reactions selected from radical polymerization reactions and hydrosilylation reactions with a silicon-bonded hydrogen atom.

10. The silicone elastomer particles according to any one of claims 7 to 9, wherein the average primary particle diameter as measured by a laser diffraction scattering method is 0.5 to 20 µm.

11. The silicone elastomer particles according to any one of claims 7 to 10, wherein the crosslinking reactive silicone composition used in forming the silicone elastomer particles is cured in a sheet form, and the JIS-A hardness measured is within a range of 10 to 80.

12. The silicone elastomer particles according to any one of claims 7 to 10, further comprising a structure in which some or all of the surface thereof is covered with one or more types selected from organopolysiloxane resins, silica, and other silicone elastomer particles.

13. The silicone elastomer particles according to any one of claims 7 to 10, further comprising a mesoporous structure.

14. The silicone elastomer particles according to any one of claims 7 to 1, further comprising an oil agent that is liquid at 40°C in the silicone elastomer particles.

15. The silicone elastomer particles according to any one of claims 7 to 14, having biodegradability.

16. The silicone elastomer particles according to any one of claims 7 to 14, wherein a divalent organic group having a partial structure formed by radical polymerization or hydrosilylation reaction of a reactive group-containing polycaprolactone compound is active with respect to biodegradable reactions, and in a biodegradable environment, a crosslinked structure formed between silicon atoms in the silicone elastomer particle is at least partially cleaved, and primary particles of the silicone elastomer particles have a property of disintegrating with the generation of a polyorganosiloxane with a non-crosslinked structure.

17. A cosmetic raw material, comprising the silicone elastomer particles according to any one of claims 7 to 16.

18. A cosmetic material composition, comprising the silicone elastomer particles according to any one of claims 7 to 16.

19. An organic resin additive, comprising the silicone elastomer particles according to any one of claims 7 to 16.

20. An organic resin, comprising the silicone elastomer particles according to any one of claims 7 to 16.

21. A method of manufacturing the silicone elastomer particles according to any one of claims 7 to 16, comprising the following step (I) and step (II).
Step (I):
a step of emulsifying in water
(A) at least one type of reactive organopolysiloxane selected from the following component (a1) and component (a2);
(a1) an organopolysiloxane having at least three organic groups containing a (meth)acryloxy group of at least one type selected from organic groups containing a methacryloxy group and organic groups containing an acryloxy group in each molecule;
(a2) an organopolysiloxane having at least three silicon atom-bonded hydrogen atoms per molecule;
(B) a reactive group-containing polycaprolactone compound according to any one of claims 1 to 4; and
(C) one or more curing agent selected from radical polymerization initiators and hydrosilylation reaction catalysts;
and forming crosslinkable silicone emulsified particles; and
Step (II):
a step of curing the crosslinking reactive silicone emulsified particle obtained in step (I) in the presence of (C) a curing agent to obtain silicone elastomer particles.

22. A method of manufacturing the silicone elastomer particles according to claim 21, wherein in step (I) at least a portion of component (A) is (a1) an organopolysiloxane having at least three organic groups containing a (meth)acryloxy group of at least one type selected from organic groups containing a methacryloxy group and organic groups containing an acryloxy group in each molecule.
